# EUROPEAN PATENT APPLICATION

(11) **EP 4 620 942 A2**
(43) Date of publication of application: **24.09.2025**
(21) Application number: 23827310.6
(22) Date of filing: 02.11.2023
(51) Int. Cl.: C07C 67/08, C07B 61/00, C07C 67/60, C07C 69/54

(54) **(METH)ACRYLIC ACID ESTER PRODUCTION METHOD**

(30) Priority: 18.11.2022 JP 2022185007
(71) Applicant: Nippon Shokubai Co., Ltd., Osaka-shi, Osaka 541-0043 (JP)
(72) Inventor: NAKAMURA, Tsukuru, Himeji-shi, Hyogo 671-1282 (JP); TAKAGAKI, Yuji, Himeji-shi, Hyogo 671-1282 (JP)
(74) Representative: Schön, Christoph
(86) International application number: PCT/JP2023/039710
(87) International publication number: WO 2023/249130

(57) **Abstract**

To provide a technique for improving the yield of a (meth)acrylic acid ester. A method of producing a (meth)acrylic acid ester, the method including: reacting (meth)acrylic acid with a secondary alcohol in a reactor in the presence of an acid-type esterification catalyst and a polymerization inhibitor; and contacting a liquid L containing an esterified product of the esterification catalyst obtained during production of the (meth)acrylic acid ester with water at a temperature T of 50°C or higher and 105°C or lower in the reactor and/or a retention tank.

## Description

### Technical Field

The present invention relates to a method of producing a (meth)acrylic acid ester. In particular, the present invention relates to a method of producing a (meth)acrylic acid ester by direct esterification between (meth)acrylic acid and a secondary alcohol.

### Background Art

It is a known technique to form a (meth)acrylic acid ester by an esterification reaction between an alcohol and (meth)acrylic acid. This reaction is an equilibrium reaction accompanied by generation of water as will be shown below.

[Chem. 1] CH₂=CH-COOH + R-OH ⇔ CH₂=CH-COOR + H₂O CH₂=C(CH₃)-COOH + R-OH ⇔ CH₂=C(CH₃)-COOR + H₂O

For shifting the equilibrium reaction toward a direction of forming a (meth)acrylic acid ester, it is necessary to remove the water formed during the reaction. On the other hand, this reaction is generally accompanied by a side reaction which forms impurities. These impurities are preferably removed from the viewpoint of providing a (meth)acrylic acid ester of high purity which meets technical requirements for its final use as a monomer for production of a polymer which can be used in many fields of application. Furthermore, for economic reasons, utilizable products present in a crude reaction mixture, in particular, an unreacted reaction material and a catalyst, are preferably recycled in the process as far as possible.

For achieving these objectives, separation/purification processes including a series of combined distillation, extraction and/or static separation are generally carried out, which are difficult to carry out particularly due to the presence of an azeotropic mixture, and are expensive in terms of energy.

For example, the specification of US 6072076 discloses a process for forming an alkyl (meth)acrylate by esterification of (meth)acrylic acid with an alkanol having a chain length in a range of 1 or more and 8 or less carbon atoms in the presence of an acid esterification catalyst.

Also, JP 2014-534972 T (corresponding to the specification of US 2015/0299093) discloses a method for continuous production of 2-octyl acrylate by direct esterification, the method including use of a single reactor and recycling of utilizable compounds, for example, on the one hand an unreacted reaction material and on the other hand an acid catalyst (in particular a sulfur-containing acid-type esterification catalyst, especially a sulfonic acid-type acid catalyst), to continuously produce 2-octyl acrylate of very high purity in a high yield.

### Summary of Invention

However, the method disclosed in the specification of US 6072076 cannot be applied in the production of a (meth)acrylic acid ester by an esterification reaction of (meth)acrylic acid with a secondary alcohol. For example, this is because the secondary alcohol is more likely to cause a dehydration reaction that leads to the formation of an alkene and water in the presence of an acid catalyst than a primary alcohol such as 2-ethylhexanol. The water formed is accumulated by reintroducing at least a part of the aqueous phase generated by the esterification reaction into the system, resulting in a risk that the target (meth)acrylic acid ester is more easily decomposed into a secondary alcohol and (meth)acrylic acid by hydrolysis. Furthermore, according to the specification of US 6072076, purification of a crude reaction mixture containing the desired (meth)acrylic acid ester and the residual alcohol is carried out by distillation, in a rectification apparatus, with a long retention time in the presence of an acid catalyst. Synthesis of a (meth)acrylic acid ester using a secondary alcohol involves a problem that an alkene and water are generated by the distillation, and that the (meth)acrylic acid ester is decomposed (that is, yield of the (meth)acrylic acid ester is low).

In addition, the technique disclosed in JP 2014-534972 T (corresponding to the specification of US 2015/0299093) involves a problem that it is difficult to sufficiently suppress decomposition of the (meth)acrylic acid ester (that is, yield of the (meth)acrylic acid ester is low).

As described above, the techniques disclosed in the specification of US 6072076 and JP 2014-534972 T (corresponding to the specification of US 2015/0299093) cannot be said to give a sufficient yield of the (meth)acrylic acid ester. Accordingly, the present invention has been made in view of the above circumstances, and an object thereof is to provide a technique for improving the yield of a (meth)acrylic acid ester.

The present inventors have conducted intensive studies to solve the above problems. As a result, the present inventors have found that the above problems can be solved by contacting a liquid containing an esterified product of an esterification catalyst obtained during production of the (meth)acrylic acid ester with water at a temperature within a predetermined range in a reactor and/or a retention tank, and have completed the present invention.

That is, the above object is achieved by a method of producing a (meth)acrylic acid ester, the method including: reacting (meth)acrylic acid with a secondary alcohol in a reactor in the presence of an acid-type esterification catalyst and a polymerization inhibitor; and contacting a liquid L containing an esterified product of the esterification catalyst obtained during production of the (meth)acrylic acid ester with water at a temperature T of 50°C or higher and 105°C or lower in the reactor and/or a retention tank.

### Brief Description of Drawings

FIG. 1 is a schematic diagram illustrating an embodiment of a (meth)acrylic acid ester production process.
FIG. 2 is a schematic diagram illustrating another embodiment of the (meth)acrylic acid ester production process.
FIG. 3 is a schematic diagram illustrating another embodiment of the (meth)acrylic acid ester production process.
FIG. 4 is a schematic diagram illustrating another embodiment of the (meth)acrylic acid ester production process.
FIG. 5 is a schematic diagram illustrating another embodiment of the (meth)acrylic acid ester production process.
FIG. 6 is a schematic diagram illustrating another embodiment of the (meth)acrylic acid ester production process.

### Description of Embodiments

The present invention provides a method of producing a (meth)acrylic acid ester, the method including: reacting (meth)acrylic acid with a secondary alcohol in a reactor in the presence of an acid-type esterification catalyst and a polymerization inhibitor; and contacting a liquid L containing an esterified product of the esterification catalyst obtained during production of the (meth)acrylic acid ester with water at a temperature T of 50°C or higher and 105°C or lower in the reactor and/or a retention tank. An object of the present invention is to provide a technique for improving the yield of a (meth)acrylic acid ester.

Hereinafter, embodiments of the present invention will be described. It should be noted that the present invention is not only limited to the following embodiments and can be variously modified within the scope of claims. The embodiments described in the present specification can be combined in any way to constitute another embodiment.

In the present specification, the contacting a liquid L containing an esterified product of the acid-type esterification catalyst obtained during production of the (meth)acrylic acid ester with water at a temperature T of 50°C or higher and 105°C or lower in the reactor and/or a retention tank is also simply referred to as "warm water treatment".

In the present specification, the "acid-type esterification catalyst" is also simply referred to as "esterification catalyst".

In the present specification, the "esterified product of the acid-type esterification catalyst" is also simply referred to as "esterified product of the esterification catalyst".

In the present specification, the term "(meth)acrylic" includes both acrylic and methacrylic. Thus, for example, the term "(meth)acrylic acid" includes both acrylic acid and methacrylic acid.

In the present specification, physical properties and the like are measured under a condition of room temperature (25 ± 5°C) unless otherwise specified.

It is to be understood that the terms used herein are used in their ordinary meanings in the art, unless otherwise specified. Accordingly, unless defined otherwise, all the technical terminologies and chemical technical terms used herein have the same meaning as commonly understood by those skilled in the art to which the present invention belongs. In case of conflict, the present specification (including definitions) is prior. Throughout the present specification, it is to be understood that the expression of a singular form includes also a concept of a plural form thereof, unless otherwise specified. Therefore, it is to be understood that an article for a singular form (for example, "a", "an", "the" and the like in the case of English) includes a concept of a plural form thereof, unless otherwise stated.

The present invention is characterized in that a liquid containing an esterified product of an esterification catalyst obtained during production of the (meth)acrylic acid ester is contacted with water at a temperature within a predetermined range in a reactor and/or a retention tank. With this configuration, the yield of the (meth)acrylic acid ester can be improved. The detailed mechanism of providing the effect is still unclear, but is considered as follows.

The present inventors have found that an esterified product of an esterification catalyst is formed during production of a (meth)acrylic acid ester, and that, when the esterified product of the esterification catalyst is introduced into a productization column and subjected to a thermal load, the esterified product is decomposed into the esterification catalyst and a secondary alcohol. The present inventors have surmised that the esterified product of the esterification catalyst is formed, for example, by a reaction of the secondary alcohol and/or an alkene formed by an intramolecular dehydration reaction of the secondary alcohol with the esterification catalyst.

In addition, the present inventors have found that the yield and purity of the (meth)acrylic acid ester tend to increase as the amount of the secondary alcohol in the product after thermal aging obtained after the thermal aging of the product containing the (meth)acrylic acid ester decreases. The present inventors have surmised that, in the productization column, the esterified product of the esterification catalyst is decomposed into the esterification catalyst and the secondary alcohol and consequently, the secondary alcohol is dehydrated by the esterification catalyst to form an alkene and water, and that the (meth)acrylic acid ester is hydrolyzed in the formed water to form the secondary alcohol and (meth)acrylic acid, thereby reducing the yield of the (meth)acrylic acid ester.

As a result of further intensive studies on the above facts and assumption, the present inventors have found that the esterified product of the esterification catalyst is decomposed by contacting a liquid containing the esterified product of the esterification catalyst obtained during the production of the (meth)acrylic acid ester with water at a temperature within a predetermined range before the (meth)acrylic acid ester is introduced into the productization column. The decomposition of the esterified product of the esterification catalyst facilitates removal of the esterification catalyst. As a result, a decomposition rate of the (meth)acrylic acid ester in the productization column is reduced, and the yield of the (meth)acrylic acid ester as the final product can be improved. It should be noted that the mechanism described above is an assumption and does not limit the technical scope of the present invention.

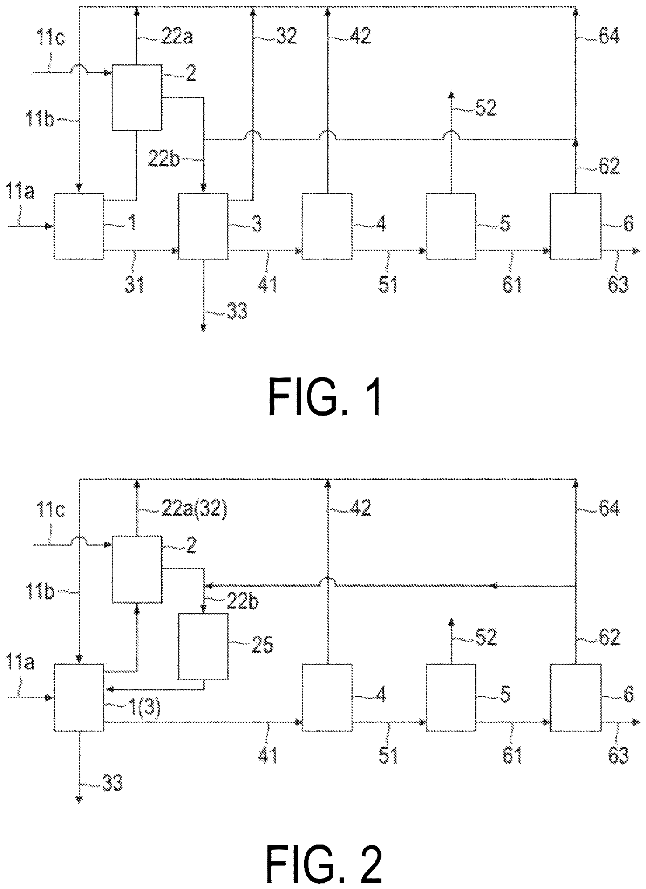

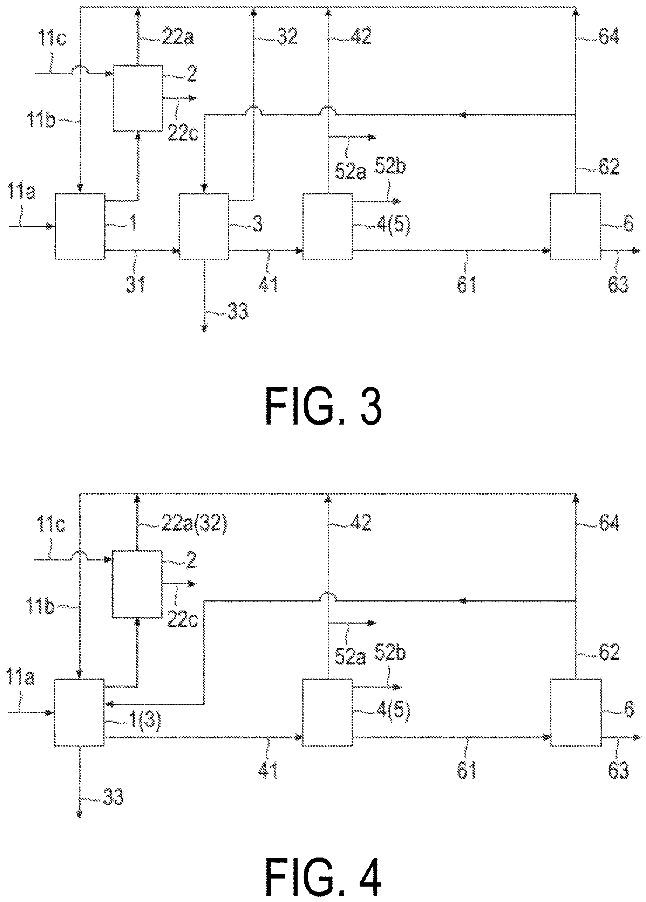

In the present specification, the term "reactor and/or retention tank" refers to a reactor, a retention tank, both of them, or a tank having the functions of a reactor and a retention tank (for example, a reactor having the function of a retention tank as illustrated in FIGS. 2 and 4 which will be described later). **In** an embodiment of the present invention, the liquid L containing the esterified product of the esterification catalyst obtained during the production of the (meth)acrylic acid ester is contacted with water at a temperature T of 50°C or higher and 105°C or lower preferably in a retention tank or a reactor having the function of a retention tank, more preferably in a retention tank.

When the liquid L containing the esterified product of the esterification catalyst obtained during the production of the (meth)acrylic acid ester (also simply referred to as "liquid L" in the present specification) is contacted with water in the reactor and/or the retention tank, the water may be in a liquid state, a gaseous state (water vapor), or a combination of the states. When the liquid L is contacted with water at a temperature within a predetermined range, the liquid L may be contacted with liquid water at a temperature exceeding 100°C, for example, in an increased pressure environment.

In an embodiment of the present invention, the method of producing a (meth)acrylic acid ester may include charging water in a liquid state, a gaseous state, or a combination of the states (that is, a part or all of water to be contacted with the liquid L) into the reactor and/or the retention tank while distilling off water (while distilling a mixture containing water).

As described above, the method of producing a (meth)acrylic acid ester according to the present invention includes contacting the liquid L with water at the temperature T of 50°C or higher and 105°C or lower in the reactor and/or the retention tank. When the temperature T is only lower than 50°C, the yield of the (meth)acrylic acid ester is insufficient. When the temperature T is only a temperature exceeding 105°C, it may be disadvantageous in terms of cost.

The temperature T is not particularly limited, but is preferably 55°C or higher and 105°C or lower, more preferably 60°C or higher and 105°C or lower, even more preferably 65°C or higher and 105°C or lower, still more preferably 70°C or higher and 105°C or lower, even still more preferably 75°C or higher and 105°C or lower, yet even still more preferably 80°C or higher and 105°C or lower, yet even still more preferably higher than 80°C and 105°C or lower, yet even still more preferably 85°C or higher and 105°C or lower, and particularly preferably 95°C or higher and 105°C or lower. By contacting the liquid L with water at the temperature T within these ranges in the reactor and/or the retention tank, the yield of the (meth)acrylic acid ester is further improved. In addition, the (meth)acrylic acid ester can be produced in a high yield in a shorter period of time.

In the present specification, the temperature T represents a "temperature when the liquid L is contacted with water in the reactor and/or the retention tank", and specifically represents a temperature of a mixture containing the liquid L and water in the reactor and/or the retention tank. The temperature of the mixture containing the liquid L and water in the reactor and/or retention tank is measured by a thermometer installed in the reactor and/or retention tank.

In the present specification, "the method of producing a (meth)acrylic acid ester includes contacting the liquid L with water at the temperature T within a predetermined range in the reactor and/or the retention tank" means "as long as the method of producing a (meth)acrylic acid ester includes contacting the liquid L with water at the temperature T within the range in the reactor and/or the retention tank, it may further include contacting the liquid L with water at a temperature outside the range in the reactor and/or retention tank".

The contact of the liquid L with water in the reactor and/or the retention tank may include contacting the liquid L with water for a holding time within a predetermined range in a state where the temperature T is held within a range of a set temperature ± 5°C, when the set temperature is a temperature in a predetermined range (in the present specification, the phrase "within a range of a set temperature ± 5°C" means "within a range of the set temperature - 5°C or higher and the set temperature + 5°C or lower"). For example, in the method of producing a (meth)acrylic acid ester according to a preferred embodiment of the present invention, the contact of the liquid L with water in the reactor and/or retention tank includes contacting the liquid L with the water for a holding time of 0.5 hours or more and 100 hours or less in a state where the temperature T is held within a range of a set temperature ± 5°C, when the set temperature is a temperature in a range of 55°C or higher and 100°C or lower (in the present specification, the phrase "within a range of a set temperature ± 5°C" means "within a range of the set temperature - 5°C or higher and the set temperature + 5°C or lower").

In the present specification, the holding time represents a "time during which the temperature (temperature T) of the mixture containing the liquid L and water is held within the range of the set temperature ± 5°C from a time point at which the temperature (temperature T) of the mixture reaches the range of the set temperature ± 5°C in the reactor and/or the retention tank as a starting point". An end point of the holding time is a time point at which the temperature T goes from within the range of the set temperature ± 5°C to outside the range.

In the present specification, when one set temperature is selected, if there are two or more periods of time during which the temperature (temperature T) of the mixture containing the liquid L and water is held within the range of the set temperature ± 5°C with a period of time during which the temperature falls outside this range being interposed therebetween, the holding time at the set temperature is treated as a total of the two or more periods of time during which the temperature is held within the range of the set temperature ± 5°C.

For example, when 90°C is selected as one set temperature, if there is only one period of time during which the temperature T is held in a range of 85°C or higher and 95°C or lower and the period of time is 10 hours, the holding time at the set temperature of 90°C is 10 hours.

For example, when 90°C is selected as one set temperature, if there are two periods of time during which the temperature T is held in the range of 85°C or higher and 95°C or lower with a period of time during which the temperature T falls outside this range being interposed therebetween, and the two periods of time during which the temperature T is held in the range of 85°C or higher and 95°C or lower are 2 hours and 8 hours, the holding time at the set temperature of 90°C is 10 hours.

The set temperature may be changed during the contact of the liquid L with water in the reactor and/or the retention tank. That is, only one set temperature may be selected, or two or more set temperatures may be selected. When the set temperature is changed, if two or more identical set temperatures are selected so that the periods of time during which the two or more identical set temperatures are set are discontinuous, the two or more identical set temperatures are treated as different set temperatures, and the holding times at the set temperatures within the predetermined temperature range are calculated.

When the liquid L is contacted with water in the reactor and/or the retention tank, if only one set temperature is selected within a predetermined temperature range, the holding time at the set temperature is the holding time at the set temperature within the predetermined temperature range.

For example, when the set temperature range is 55°C or higher and 100°C or lower, only one temperature is selected as the set temperature in the contact of the liquid L with water in the reactor and/or the retention tank, and the set temperature is 90°C, if the holding time at the set temperature of 90°C (the time at 85°C or higher and 95°C or lower, that is, the time during which the temperature T is 85°C or higher and 95°C or lower) is 10 hours, the holding time at the set temperature in the range of 55°C or higher and 100°C or lower is 10 hours.

In the present specification, when the set temperature is changed during the contact of the liquid L with water in the reactor and/or the retention tank and two or more temperatures are selected as the set temperature, the holding time at the set temperature within the predetermined temperature range is treated as follows.

When the set temperature is changed, if two or more set temperatures are selected within a predetermined temperature range, a total of the holding times at the respective selected set temperatures is treated as the holding time at the set temperature within the predetermined temperature range. When the set temperature is changed, if one or more set temperatures are selected within a predetermined temperature range and one or more set temperatures are selected outside the predetermined temperature range, the total of the holding times at the respective set temperatures selected within the predetermined temperature range (when the time is only one, the time) is treated as the holding time at the set temperature within the predetermined temperature range.

The starting point and end point of the holding time at each set temperature are the same as the starting point and end point of the holding time described above. However, a part or all of the time from a time point at which the set temperature is changed to a time point at which the temperature T first goes from within the range of the set temperature before change ± 5°C to outside the range and a part or all of the holding time at another set temperature overlap in some cases. In a case where the overlapping time exists, the holding time at the set temperature within a predetermined temperature range is treated as a value obtained by subtracting the overlapping time from the total of the holding times at the respective set temperatures within the predetermined temperature range.

The holding time (holding time at the set temperature within a predetermined temperature range) is not particularly limited, but is preferably 0.1 hours or more, more preferably 0.5 hours or more, even more preferably 1 hour or more, still more preferably 3 hours or more, even still more preferably 5 hours or more, and particularly preferably 10 hours or more. Within this range, the yield of the (meth)acrylic acid ester is further improved. The holding time at the set temperature within the predetermined temperature range is not particularly limited, but is preferably 100 hours or less, more preferably 70 hours or less, even more preferably 50 hours or less, still more preferably 40 hours or less, even still more preferably 30 hours or less, yet even still more preferably 20 hours or less, and particularly preferably 10 hours or less. Within these ranges, the (meth)acrylic acid ester can be produced in a shorter period of time. Examples of the range of the holding time in a preferred embodiment include any combination of upper and lower limits selected from these upper and lower limits. Examples of the holding time in a preferred embodiment include 0.1 hours or more and 100 hours or less, 0.5 hours or more and 100 hours or less, 1 hour or more and 100 hours or less, 3 hours or more and 100 hours or less, 5 hours or more and 100 hours or less, 10 hours or more and 100 hours or less, 1 hour or more and 70 hours or less, 3 hours or more and 70 hours or less, 5 hours or more and 70 hours or less, 10 hours or more and 70 hours or less, 1 hour or more and 50 hours or less, 3 hours or more and 50 hours or less, 5 hours or more and 10 hours or less, 10 hours or more and 50 hours or less, 1 hour or more and 40 hours or less, 3 hours or more and 40 hours or less, 5 hours or more and 10 hours or less, 10 hours or more and 40 hours or less, 1 hour or more and 30 hours or less, 3 hours or more and 30 hours or less, 5 hours or more and 30 hours or less, 10 hours or more and 30 hours or less, 1 hour or more and 20 hours or less, 3 hours or more and 20 hours or less, 5 hours or more and 20 hours or less, 1 hour or more and 10 hours or less, 3 hours or more and 10 hours or less, and 5 hours or more and 10 hours or less, but the holding time is not limited thereto.

The set temperature is preferably a temperature within a range of 50°C or higher and 100°C or lower, more preferably a temperature within a range of 55°C or higher and 100°C or lower, even more preferably a temperature within a range of 60°C or higher and 100°C or lower, still more preferably a temperature within a range of 65°C or higher and 100°C or lower, even still more preferably a temperature within a range of 70°C or higher and 100°C or lower, yet even still more preferably a temperature within a range of 75°C or higher and 100°C or lower, yet even still more preferably a temperature within a range of higher than 75°C and 100°C or lower, yet even still more preferably a temperature within a range of 80°C or higher and 100°C or lower, yet even still more preferably a temperature within a range of higher than 80°C and 100°C or lower, yet even still more preferably a temperature within a range of 85°C or higher and 100°C or lower, yet even still more preferably in the range of 90°C or higher and 100°C or lower, and particularly preferably in the range of 95°C or higher and 100°C or lower. When the temperature within these ranges is set as the set temperature, the yield of the (meth)acrylic acid ester is further improved by contacting the liquid L with water in the reactor and/or the retention tank in a state where the temperature T is held within the range of the set temperature ± 5°C. In addition, the (meth)acrylic acid ester can be produced in a high yield in a shorter period of time.

It is preferable to select only one set temperature. Only one holding time is preferred.

As described above, the contact of the liquid L with water in the reactor and/or the retention tank may include contacting the liquid L with water for a holding time within a predetermined range in a state where the temperature T is held within a range of the set temperature ± 5°C (within a range of the set temperature - 5°C or higher and the set temperature + 5°C or lower), when the set temperature is a temperature in a predetermined range. For example, in a preferred embodiment of the present invention, the contact of the liquid L with water in the reactor and/or retention tank in the method of producing a (meth)acrylic acid ester includes contacting the liquid L with water for a holding time of 0.5 hours or more and 100 hours or less in a state where the temperature T is held within a range of a set temperature ± 5°C (within a range of the set temperature - 5°C or higher and the set temperature + 5°C or lower), when the set temperature is a temperature in a range of 55°C or higher and 100°C or lower. These embodiments may further include selecting a set temperature outside these temperature ranges and contacting the liquid L with water, as long as they include selecting a set temperature within these temperature ranges and contacting the liquid L with water.

Examples of a preferred combination of the set temperature and the holding time include a combination of a set temperature in a range of 50°C or higher and 100°C or lower and a holding time of 0.1 hours or more and 100 hours or less in a state where the temperature T is held within a range of the set temperature ± 5°C; a combination of a set temperature in a range of 55°C or higher and 100°C or lower and a holding time of 0.1 hours or more and 70 hours or less in a state where the temperature T is held within a range of the set temperature ± 5°C; a combination of a set temperature in a range of 55°C or higher and 100°C or lower and a holding time of 0.5 hours or more and 70 hours or less in a state where the temperature T is held within a range of the set temperature ± 5°C; a combination of a set temperature in a range of 55°C or higher and 100°C or lower and a holding time of 1 hour or more and 70 hours or less in a state where the temperature T is held within a range of the set temperature ± 5°C; a combination of a set temperature in a range of 55°C or higher and 100°C or lower and a holding time of 5 hours or more and 70 hours or less in a state where the temperature T is held within a range of the set temperature ± 5°C; a combination of a set temperature in a range of 60°C or higher and 100°C or lower and a holding time of 0.1 hours or more and 70 hours or less in a state where the temperature T is held within a range of the set temperature ± 5°C; a combination of a set temperature in a range of 60°C or higher and 100°C or lower and a holding time of 0.5 hours or more and 70 hours or less in a state where the temperature T is held within a range of the set temperature ± 5°C; a combination of a set temperature in a range of 60°C or higher and 100°C or lower and a holding time of 5 hours or more and 70 hours or less in a state where the temperature T is held within a range of the set temperature ± 5°C; a combination of a set temperature in a range of 65°C or higher and 100°C or lower and a holding time of 0.1 hours or more and 70 hours or less in a state where the temperature T is held within a range of the set temperature ± 5°C; a combination of a set temperature in a range of 65°C or higher and 100°C or lower and a holding time of 0.5 hours or more and 70 hours or less in a state where the temperature T is held within a range of the set temperature ± 5°C; a combination of a set temperature in a range of 65°C or higher and 100°C or lower and a holding time of 1 hour or more and 70 hours or less in a state where the temperature T is held within a range of the set temperature ± 5°C; a combination of a set temperature in a range of 65°C or higher and 100°C or lower and a holding time of 0.1 hours or more and 50 hours or less in a state where the temperature T is held within a range of the set temperature ± 5°C; a combination of a set temperature in a range of 65°C or higher and 100°C or lower and a holding time of 0.5 hours or more and 50 hours or less in a state where the temperature T is held within a range of the set temperature ± 5°C; a combination of a set temperature in a range of 65°C or higher and 100°C or lower and a holding time of 3 hours or more and 50 hours or less in a state where the temperature T is held within a range of the set temperature ± 5°C; a combination of a set temperature in a range of 65°C or higher and 100°C or lower and a holding time of 5 hours or more and 70 hours or less in a state where the temperature T is held within a range of the set temperature ± 5°C; a combination of a set temperature in a range of 70°C or higher and 100°C or lower and a holding time of 0.1 hours or more and 50 hours or less in a state where the temperature T is held within a range of the set temperature ± 5°C; a combination of a set temperature in a range of 70°C or higher and 100°C or lower and a holding time of 0.5 hours or more and 50 hours or less in a state where the temperature T is held within a range of the set temperature ± 5°C; a combination of a set temperature in a range of 70°C or higher and 100°C or lower and a holding time of 5 hours or more and 50 hours or less in a state where the temperature T is held within a range of the set temperature ± 5°C; a combination of a set temperature in a range of 75°C or higher and 100°C or lower and a holding time of 0.1 hours or more and 40 hours or less in a state where the temperature T is held within a range of the set temperature ± 5°C; a combination of a set temperature in a range of 75°C or higher and 100°C or lower and a holding time of 1 hour or more and 40 hours or less in a state where the temperature T is held within a range of the set temperature ± 5°C; a combination of a set temperature in a range of 75°C or higher and 100°C or lower and a holding time of 3 hours or more and 40 hours or less in a state where the temperature T is held within a range of the set temperature ± 5°C; a combination of a set temperature in a range of 75°C or higher and 100°C or lower and a holding time of 5 hours or more and 40 hours or less in a state where the temperature T is held within a range of the set temperature ± 5°C; a combination of a set temperature in a range of higher than 75°C and 100°C or lower and a holding time of 0.1 hour or more and 40 hours or less in a state where the temperature T is held within a range of the set temperature ± 5°C; a combination of a set temperature in a range of higher than 75°C and 100°C or lower and a holding time of 1 hour or more and 40 hours or less in a state where the temperature T is held within a range of the set temperature ± 5°C; a combination of a set temperature in a range of higher than 75°C and 100°C or lower and a holding time of 5 hours or more and 40 hours or less in a state where the temperature T is held within a range of the set temperature ± 5°C; a combination of a set temperature in a range of 80°C or higher and 100°C or lower and a holding time of 0.1 hours or more and 40 hours or less in a state where the temperature T is held within a range of the set temperature ± 5°C; a combination of a set temperature in a range of 80°C or higher and 100°C or lower and a holding time of 1 hour or more and 40 hours or less in a state where the temperature T is held within a range of the set temperature ± 5°C; a combination of a set temperature in a range of 80°C or higher and 100°C or lower and a holding time of 5 hours or more and 40 hours or less in a state where the temperature T is held within a range of the set temperature ± 5°C; a combination of a set temperature in a range of higher than 80°C and 100°C or lower and a holding time of 1 hour or more and 40 hours or less in a state where the temperature T is held within a range of the set temperature ± 5°C; a combination of a set temperature in a range of higher than 80°C and 100°C or lower and a holding time of 3 hours or more and 40 hours or less in a state where the temperature T is held within a range of the set temperature ± 5°C; a combination of a set temperature in a range of higher than 80°C and 100°C or lower and a holding time of 1 hour or more and 30 hours or less in a state where the temperature T is held within a range of the set temperature ± 5°C; a combination of a set temperature in a range of higher than 80°C and 100°C or lower and a holding time of 5 hours or more and 30 hours or less in a state where the temperature T is held within a range of the set temperature ± 5°C; a combination of a set temperature in a range of 85°C or higher and 100°C or lower and a holding time of 1 hour or more and 40 hours or less in a state where the temperature T is held within a range of the set temperature ± 5°C; a combination of a set temperature in a range of 85°C or higher and 100°C or lower and a holding time of 3 hours or more and 40 hours or less in a state where the temperature T is held within a range of the set temperature ± 5°C; a combination of a set temperature in a range of 85°C or higher and 100°C or lower and a holding time of 1 hour or more and 30 hours or less in a state where the temperature T is held within a range of the set temperature ± 5°C; a combination of a set temperature in a range of 85°C or higher and 100°C or lower and a holding time of 5 hours or more and 30 hours or less in a state where the temperature T is held within a range of the set temperature ± 5°C; a combination of a set temperature in a range of 90°C or higher and 100°C or lower and a holding time of 1 hour or more and 30 hours or less in a state where the temperature T is held within a range of the set temperature ± 5°C; a combination of a set temperature in a range of 90°C or higher and 100°C or lower and a holding time of 5 hours or more and 30 hours or less in a state where the temperature T is held within a range of the set temperature ± 5°C; a combination of a set temperature in a range of 95°C or higher and 100°C or lower and a holding time of 1 hour or more and 20 hours or less in a state where the temperature T is held within a range of the set temperature ± 5°C; a combination of a set temperature in a range of 95°C or higher and 100°C or lower and a holding time of 3 hours or more and 20 hours or less in a state where the temperature T is held within a range of the set temperature ± 5°C; a combination of a set temperature in a range of 95°C or higher and 100°C or lower and a holding time of 5 hours or more and 20 hours or less in a state where the temperature T is held within a range of the set temperature ± 5°C; a combination of a set temperature in a range of 95°C or higher and 100°C or lower and a holding time of 1 hour or more and 10 hours or less in a state where the temperature T is held within a range of the set temperature ± 5°C; a combination of a set temperature in a range of 95°C or higher and 100°C or lower and a holding time of 3 hours or more and 10 hours or less in a state where the temperature T is held within a range of the set temperature ± 5°C; and a combination of a set temperature in a range of 95°C or higher and 100°C or lower and a holding time of 5 hours or more and 10 hours or less in a state where the temperature T is held within a range of the set temperature ± 5°C, but the combination of the set temperature and the holding time is not limited thereto. The contact of the liquid L with water in the reactor and/or the retention tank may further include selecting a set temperature outside these temperature ranges and contacting the liquid L with water, as long as the contact includes selecting a set temperature within these temperature ranges and contacting the liquid L with water.

In an embodiment of the present invention, the contact of the liquid L with water in the reactor and/or the retention tank in the method of producing a (meth)acrylic acid ester preferably includes contacting the liquid L with water for 0.1 hours or more in a state where the temperature T is held in a range of 50°C or higher and 105°C or lower. In the present embodiment, the temperature range in which the temperature T is held is more preferably 55°C or higher and 105°C or lower, even more preferably 65°C or higher and 105°C or lower, still more preferably 75°C or higher and 105°C or lower, even still more preferably higher than 80°C and 105°C or lower, yet even still more preferably 85°C or higher and 105°C or lower, and particularly preferably 95°C or higher and 105°C or lower. In the present embodiment, the time during which the temperature T is held is more preferably 0.5 hours or more, and even more preferably 3 hours or more.

In an embodiment of the present invention, the contact of the liquid L with water in the reactor and/or the retention tank in the method of producing a (meth)acrylic acid ester preferably includes at least one selected from the group consisting of the following (i), (ii), (iii), (iv) and (v), more preferably includes at least one selected from the group consisting of the following (iii), (iv) and (v), even more preferably includes at least one selected from the group consisting of the following (iv) and (v), and particularly preferably includes the following (v):
(i) contacting the liquid L with the water for 5 hours or more and 70 hours or less in a state where the temperature T is held in a range of 55°C or higher and 65°C or lower;
(ii) contacting the liquid L with the water for 5 hours or more and 100 hours or less in a state where the temperature T is held in a range of 65°C or higher and 75°C or lower;
(iii) contacting the liquid L with the water for 0.5 hours or more and 40 hours or less in a state where the temperature T is held in a range of 75°C or higher and 85°C or lower;
(iv) contacting the liquid L with the water for 0.5 hours or more and 25 hours or less in a state where the temperature T is held in a range of 85°C or higher and 95°C or lower; and
(v) contacting the liquid L with the water for 0.1 hours or more and 10 hours or less in a state where the temperature T is held in a range of 95°C or higher and 105°C or lower.

In the case of (i) above, the time (the time during which the liquid L is contacted with the water in a state where the temperature T is held in the range of 55°C or higher and 65°C or lower) is preferably 45 hours or more and 70 hours or less. In the case of (ii) above, the time (the time during which the liquid L is contacted with the water in a state where the temperature T is held in the range of 65°C or higher and 75°C or lower) is preferably 35 hours or more and 45 hours or less. In the case of (iii) above, the time (the time during which the liquid L is contacted with the water in a state where the temperature T is held in the range of 75°C or higher and 85°C or lower) is preferably 20 hours or more and 40 hours or less, more preferably 25 hours or more and 35 hours or less. In the case of (iv) above, the time (the time during which the liquid L is contacted with the water in a state where the temperature T is held in the range of 85°C or higher and 95°C or lower) is preferably 10 hours or more and 25 hours or less. In the case of (vi) above, the time (the time during which the liquid L is contacted with the water in a state where the temperature T is held in the range of 95°C or higher and 105°C or lower) is preferably 0.5 hours or more and 10 hours or less, more preferably 1 hour or more and 10 hours or less, even more preferably 3 hours or more and 10 hours or less, and particularly preferably 5 hours or more and 10 hours or less.

In these embodiments, the contact of the liquid L with water in the reactor and/or retention tank may further include contacting the liquid L with water at a temperature T falling outside these temperature ranges, as long as the contact includes contacting the liquid L with water in a state where the temperature T is held within these temperature ranges.

When these relationships are satisfied, the (meth)acrylic acid ester can be produced in a high yield in a shorter period of time.

When the temperature in a predetermined range is set as the set temperature, a proportion of the holding time (the holding time when the temperature within the predetermined range is set as the set temperature) to a time (hereinafter also referred to as "elapsed time") from a time point at which a temperature (temperature T) of a mixture containing the liquid L and water first reaches a range of the set temperature ± 5°C as a starting point to a time point at which the temperature T lastly goes from within the range of the set temperature ± 5°C to outside the range of the set temperature ± 5°C as an end point, in the reactor and/or the retention tank, is not particularly limited. However, the proportion of the holding time to the elapsed time is preferably 80% or more, more preferably 90% or more, and even more preferably 95% or more (upper limit: 100%). When the holding time is within the range of the proportion, the warm water treatment can be efficiently performed.

When the set temperature is changed and two or more set temperatures are selected within the predetermined temperature range, the time from a time point at which the temperature T first reaches a range of the (set temperature first set within the predetermined temperature range) ± 5°C as the starting point, to a time point at which the temperature T lastly goes from within a range of the (set temperature lastly set within the predetermined temperature range) ± 5°C to outside the range as the end point is treated as the elapsed time.

A proportion of a mass of water to a total mass of the liquid L and water to be contacted in the reactor and/or the retention tank is not particularly limited, but is preferably 10 mass% or more, more preferably 15 mass% or more, even more preferably 20 mass% or more, still more preferably 25 mass% or more, even still more preferably 30 mass% or more, and particularly preferably 40 mass% or more (upper limit: less than 100 mass%). The proportion of the mass of water to the total mass of the liquid L and water to be contacted in the reactor and/or the retention tank is not particularly limited, but is preferably 90 mass% or less, more preferably 85 mass% or less, even more preferably 80 mass% or less, and particularly preferably 60 mass% or less (lower limit: more than 0 mass%). The proportion of the mass of water to the total mass of the liquid L and water to be contacted in the reactor and/or the retention tank may be, for example, 50 mass% or less, or 40 mass% or less. Within these ranges, the decomposition rate of the esterified product of the esterification catalyst is improved. In addition, the yield of the (meth)acrylic acid ester is further improved. In addition, since the predetermined decomposition rate of the esterified product of the esterification catalyst is reached in a shorter time, productivity of the (meth)acrylic acid ester is further improved. Accordingly, preferred examples of the proportion of the mass of water to the total mass of the liquid L and water include 10 mass% or more and 90 mass% or less, 15 mass% or more and 85 mass% or less, 20 mass% or more and 80 mass% or less, 25 mass% or more and 80 mass% or less, 30 mass% or more and 80 mass% or less, 20 mass% or more and 60 mass% or less, 25 mass% or more and 60 mass% or less, 30 mass% or more and 60 mass% or less, 40 mass% or more and 60 mass% or less, 30 mass% or more and 50 mass% or less, and 30 mass% or more and 40 mass%, but the proportion of the mass of water to the total mass of the liquid L and water is not limited thereto.

A flash point of the produced (meth)acrylic acid ester is not particularly limited. However, for example, when 1-methylheptyl acrylate is produced, a flash point of the produced 1-methylheptyl acrylate is preferably 90°C or higher, more preferably 92°C or higher, even more preferably 93°C or higher, and particularly preferably higher than 93°C (upper limit: the flash point of pure 1-methylheptyl acrylate). The flash point of the (meth)acrylic acid ester can be determined by the Cleveland open-cup method.

The purity of the produced (meth)acrylic acid ester is preferably as high as possible, and is particularly preferably 99.9 mass% or more (upper limit: 100 mass%). That is, the method of producing a (meth)acrylic acid ester according to a preferred embodiment of the present invention can produce a (meth)acrylic acid ester having a purity of 99.9 mass% or more. For example, when the (meth)acrylic acid ester is 1-methylheptyl acrylate or 1-methylheptyl methacrylate, the purity of the (meth)acrylic acid ester is preferably 99.9 mass% or more, more preferably 99.92 mass% or more, and even more preferably 99.94 mass% or more (upper limit: 100 mass%).

In an embodiment of the present invention, the method of producing 1-methylheptyl acrylate includes contacting a liquid L containing an esterified product of an esterification catalyst obtained during production of 1-methylheptyl acrylate with water at a temperature T of 50°C or higher and 105°C or lower in a reactor and/or a retention tank, and then removing a low-boiling-point substance (e.g., a substance having a boiling point lower than that of 1-methylheptyl acrylate) and a high-boiling-point substance (e.g., a substance having a boiling point higher than that of 1-methylheptyl acrylate) by purification. When the liquid L is a liquid obtained by subjecting a reaction mixture containing 1-methylheptyl acrylate and the esterified product of the esterification catalyst to a neutralization treatment and a water washing treatment, and, if necessary, an organic solvent cutting treatment (for example, a toluene cutting treatment), the purity of 1-methylheptyl acrylate can be calculated by, for example, the following equation. Here, the liquid L is preferably a liquid obtained by subjecting a reaction mixture containing **1-**methylheptyl acrylate and the esterified product of the esterification catalyst to a neutralization treatment, a water washing treatment, and an organic solvent cutting treatment (for example, a toluene cutting treatment). PURITY OF 1-METHYLHEPTYL ACRYLATE (mass%) = 100 - (TOTAL CONTENT OF 2-OCTENE, 2-OCTANOL AND ACID COMPONENT IN 1-METHYLHEPTYL ACRYLATE (mass%))

In the calculation of the above equation, a content of 2-octene in 1-methylheptyl acrylate and a content of 2-octanol in 1-methylheptyl acrylate can be quantified using gas chromatography (GC). An acid component in 1-methylheptyl acrylate can be quantified by neutralization titration.

The decomposition rate of the esterified product of the esterification catalyst by contacting the liquid L with water at the temperature T in the reactor and/or the retention tank is not particularly limited, but is preferably as high as possible from the viewpoint of the yield and purity of the (meth)acrylic acid ester.

In the method of producing a (meth)acrylic acid ester according to the present invention, details of, for example, the types and amounts of (meth)acrylic acid, secondary alcohol, acid-type esterification catalyst, and polymerization inhibitor, and organic solvent to be added if necessary, which are used in synthesis of the (meth)acrylic acid ester, are the same as those in the description of step (a) which will be described below. In addition, details of the reaction such as conditions for the reaction of the (meth)acrylic acid ester in the method of producing a (meth)acrylic acid ester according to the present invention are the same as those in the description of the step (a) which will be described below.

Hereinafter, an embodiment of the method of producing a (meth)acrylic acid ester according to the present invention will be described with reference to FIGS. 1 to 6. However, the method of producing a (meth)acrylic acid ester according to the present invention is not limited to the following embodiments.

An embodiment of the present invention is a method of producing a (meth)acrylic acid ester including the following (a), (b), (c), (d) and (e). A schematic diagram of a production process of the present embodiment is illustrated as FIG. 1.

(a) Supplying an acid-type esterification catalyst, (meth)acrylic acid, a secondary alcohol, and a polymerization inhibitor (and other components (such as an organic solvent) if used) to a reactor 1 to react the (meth)acrylic acid with the secondary alcohol in the presence of the esterification catalyst and the polymerization inhibitor, simultaneously distilling off water formed by the esterification reaction from a column top part of a first distillation column 2 as an azeotropic composition with the secondary alcohol and/or the organic solvent if used (the secondary alcohol, the organic solvent if used, or a combination thereof), condensing the resulting distillate, and allowing it to stand to separate it into an oil phase and an aqueous phase, while obtaining a reaction mixture A containing a (meth)acrylic acid ester and an esterified product of the esterification catalyst (an esterified product of the acid-type esterification catalyst) in the reactor 1 (step (a));
(b) withdrawing the reaction mixture A from a bottom of the reactor 1, supplying the reaction mixture A to a retention tank 3, subjecting the reaction mixture A to neutralization (neutralization treatment) and water washing (water washing treatment) in the retention tank 3 to obtain a mixture M1 (mixture after neutralization and water washing), separating the mixture M1 into an oil phase O1 and an aqueous phase W1, removing the aqueous phase W1 from the retention tank 3 to leave the oil phase O1 in the retention tank 3, and optionally subjecting the oil phase O1 to a heating treatment, an organic solvent cutting treatment or a combination thereof, retaining, in the retention tank 3, the oil phase (the oil phase O1, or an oil phase O1 that has been subjected to a heating treatment, an organic solvent cutting treatment, or a combination thereof) (here, the oil phase corresponds to the liquid L since it contains the esterified product of the acid-type esterification catalyst) and water while contacting them at a temperature T of 50°C or higher and 105°C or lower to obtain a mixture M2 (mixture after warm water treatment), separating the mixture M2 into an oil phase O2 and an aqueous phase W2, removing the aqueous phase W2 from the retention tank 3, optionally subjecting the oil phase O2 to a heating treatment, an organic solvent cutting treatment, or a combination thereof, collecting a gas and/or a condensate liquid obtained optionally, from the retention tank 3 (collecting a gas, a condensate liquid, or a combination thereof obtained optionally, from the retention tank 3), and obtaining a residue (the oil phase O2, or an oil phase O2 that has been subjected to a heating treatment, an organic solvent cutting treatment, or a combination thereof) in the retention tank 3 as a reaction mixture B (step (b));
(c) supplying the reaction mixture B to a second distillation column 4 and distilling it to separate it into a column bottom liquid of the second distillation column 4 in which a (meth) acrylic acid ester is contained and a recovered alcohol (step (c));
(d) withdrawing the column bottom liquid of the second distillation column 4 from a column bottom part of the second distillation column 4, supplying it to a third distillation column 5, distilling it to separate it into a purified (meth) acrylic acid ester as a final product and a column bottom liquid of the third distillation column 5, and withdrawing the purified (meth) acrylic acid ester from a column top part of the third distillation column 5 (step (d)); and
(e) withdrawing the column bottom liquid of the third distillation column 5 from a column bottom part of the third distillation column 5, supplying it to a treatment apparatus 6, and separating it into a recovered (meth)acrylic acid ester and waste oil (step (e)).

In the present embodiment, a known method can be used in the same manner or in an appropriately modified manner, and the present embodiment is not limited to the following embodiment.

### Step (a)

In this step, (meth)acrylic acid, a secondary alcohol, an acid-type esterification catalyst, and a polymerization inhibitor, and, if necessary, an organic solvent (reaction materials) are supplied to the reactor 1 via a pipe 11a. A compound other than these may be further supplied to the reactor 1. In FIG. 1, (meth)acrylic acid, a secondary alcohol, an acid-type esterification catalyst, and a polymerization inhibitor, and, if necessary, an organic solvent are supplied via the same pipe 11a, but may be supplied via another pipe. Particularly, the (meth)acrylic acid may be directly introduced into the reactor via the pipe 11a, and the acid-type esterification catalyst and the polymerization inhibitor may be directly introduced into the reactor via another pipe (not illustrated). A part of the secondary alcohol may be directly introduced into the reactor via a pipe and another part be introduced into the column top part of the first distillation column 2 via another pipe 11c in order to ensure column reflux. Alternatively, as will be described in detail below, the recovered alcohol (secondary alcohol-rich fluid) obtained from the second distillation column 4 (the subsequent purification stage) may be supplied to the reactor 1 via a pipe 11b. Alternatively, as will be described in detail below, the recovered (meth)acrylic acid ester obtained from the treatment apparatus 6 may be supplied to the reactor 1 via a pipe 64 and the pipe 11b.

Here, examples of the acid-type esterification catalyst (esterification catalyst) include a sulfur-containing acid-type esterification catalyst and an acidic cation exchange resin. Among these, as the sulfur-containing acid-type esterification catalyst, for example, a sulfur-containing acid compound such as sulfuric acid or an organic sulfonic acid can be used. Examples of the sulfur-containing acid compound include sulfuric acid, para-toluenesulfonic acid, methanesulfonic acid, benzenesulfonic acid, dodecylsulfonic acid, and xylenesulfonic acid. The acidic cation exchange resin is not limited by resin physical properties such as a resin structure, a degree of crosslinking, or a combination thereof, and, for example, a porous- or gel-type strongly acidic cation exchange resin, a weakly acidic cation exchange resin, or the like can be used, but a porous- or gel-type strongly acidic cation exchange resin can be preferably used. Examples of the porous-type strongly acidic cation exchange resin include MSC-1 (available from Dow Inc.), PK-208, PK-212, PK-216, PK-220, and PK-228 (all available from Mitsubishi Chemical Corporation), Amberlyst (trade name)-16, IR-116, IR-118, IR-122, C-26, C-26TR, C-264, and C-265 (all available from Rohm and Haas Company), SPC-108 and SPC-112 (all available from Bayer AG), and KC-470 (available from Sumitomo Chemical Co., Ltd.). Examples of the gel-type strongly acidic cation exchange resin include HCR-S, HCR-W2, and HGR-W2 (all available from Dow Inc.), SK-1B, SK-106, and SK-110 (all available from Mitsubishi Chemical Corporation), Duolite (trade name) C20H and C255LFH (all available from Rohm and Haas Company), and K1221, and K1431 (all available from Bayer AG). These esterification catalysts may be used alone, or two or more thereof may be used in combination. Among these, in terms of handleability and cost, the acid-type esterification catalyst is preferably a sulfur-containing acid-type esterification catalyst, and sulfuric acid, para-toluenesulfonic acid, and methanesulfonic acid are preferred. The acid-type esterification catalyst preferably includes a sulfur-containing acid-type esterification catalyst, more preferably includes a sulfur-containing acid compound, even more preferably includes at least one compound selected from the group consisting of sulfuric acid and an organic sulfonic acid, still more preferably includes at least one compound selected from the group consisting of sulfuric acid, para-toluenesulfonic acid, methanesulfonic acid, benzenesulfonic acid, dodecylsulfonic acid and xylenesulfonic acid, and particularly preferably includes at least one compound selected from the group consisting of sulfuric acid, para-toluenesulfonic acid and methanesulfonic acid. The acid-type esterification catalyst is preferably at least one selected from the group consisting of a sulfur-containing acid-type esterification catalyst and an acidic cation exchange resin, more preferably a sulfur-containing acid-type esterification catalyst, even more preferably a sulfur-containing acid compound, still more preferably at least one compound selected from the group consisting of sulfuric acid and an organic sulfonic acid, even still more preferably at least one compound selected from the group consisting of sulfuric acid, para-toluenesulfonic acid, methanesulfonic acid, benzenesulfonic acid, dodecylsulfonic acid, and xylenesulfonic acid, and particularly preferably at least one compound selected from the group consisting of sulfuric acid, para-toluenesulfonic acid, and methanesulfonic acid. An amount of the esterification catalyst to be added is, for example, a proportion of 0.5 parts by mass or more and 10 parts by mass or less, and preferably 1 part by mass or more and 5 parts by mass or less relative to 100 parts by mass of (meth)acrylic acid. When two or more esterification catalysts are used in combination, the amount of the esterification catalyst to be added refers to a total amount of these esterification catalysts.

The acid-type esterification catalyst (esterification catalyst), in particular, the sulfur-containing acid-type esterification catalyst, may be supplied to the reactor as it is or supplied to the reactor in the form of a solution (e.g. an aqueous solution). When the acid-type esterification catalyst (esterification catalyst) is supplied to the reactor in the form of a solution (for example, an aqueous solution), a concentration of the esterification catalyst in the solution can be, for example, 50 mass% or more and 90 mass% or less, and preferably 60 mass% or more and 80 mass% or less, but is not limited to these ranges. When two or more esterification catalysts are used in combination, the concentration of the esterification catalyst refers to a total concentration of these esterification catalysts in the solution.

Acrylic acid or methacrylic acid used as a starting material is produced by a known method, and, for example, is industrially produced from propylene or isobutene. Independently of use of a renewable alcohol, the method of producing a (meth)acrylic acid ester according to an embodiment of the present invention may extend to use of a renewable (meth)acrylic acid during the esterification. For example, acrylic acid can also be obtained by a method including a first stage of dehydration of glycerol to obtain acrolein from glycerol, followed by a stage of gas-phase oxidation of the acrolein obtained; or by dehydration of 2-hydroxypropionic acid (lactic acid) or 3-hydroxypropionic acid and esters thereof, but the method of producing (meth)acrylic acid is not limited to these methods. The (meth)acrylic acid used as a starting material preferably includes at least one compound selected from the group consisting of acrylic acid and methacrylic acid. The (meth)acrylic acid may contain acrylic acid or may include methacrylic acid, but more preferably includes methacrylic acid. Among them, acrylic acid or methacrylic acid is preferred. The (meth)acrylic acid is preferably acrylic acid or methacrylic acid, and particularly preferably methacrylic acid. An amount of the (meth)acrylic acid to be added is, for example, 10 parts by mass or more and 50 parts by mass or less and preferably 20 parts by mass or more and 35 parts by mass or less relative to 100 parts by mass of a total amount of the reaction materials (a total amount of the (meth)acrylic acid, the secondary alcohol, the acid-type esterification catalyst, and the polymerization inhibitor, and the organic solvent and/or water (the organic solvent, water, or a combination thereof) when added). When the (meth)acrylic acid is added in portions, the amount of the (meth)acrylic acid to be added refers to a total amount of the (meth)acrylic acid. When acrylic acid and methacrylic acid are used in combination, the amount of the (meth)acrylic acid to be added refers to a total amount of acrylic acid and methacrylic acid.

The secondary alcohol is not particularly limited, and examples thereof include isopropanol, 2-pentanol, 3-pentanol, 2-heptanol, 3-heptanol, 2-octanol, 3-octanol, 4-octanol, 2-nonanol, 2-decanol, 2-undecanol, 2-dodecanol, 2-tridecanol, 2-tetradecanol, 2-pentadecanol, 2-hexadecanol, 2-heptadecanol, 2-octadecanol, 2-nonadecanol, 2-eicosanol, and 2-docosanol. These secondary alcohols may be used alone, or two or more thereof may be used in combination. The secondary alcohol preferably includes at least one compound selected from the group consisting of isopropanol, 2-pentanol, 3-pentanol, 2-heptanol, 3-heptanol, 2-octanol, 3-octanol, 4-octanol, 2-nonanol, 2-decanol, 2-undecanol, 2-dodecanol, 2-tridecanol, 2-tetradecanol, 2-pentadecanol, 2-hexadecanol, 2-heptadecanol, 2-octadecanol, 2-nonadecanol, 2-eicosanol, and 2-docosanol, more preferably includes at least one compound selected from the group consisting of 2-heptanol, 3-heptanol, 2-octanol, 3-octanol, 4-octanol, and 2-nonanol, and even more preferably includes 2-octanol. Among them, at least one selected from the group consisting of 2-octanol, 3-octanol and 4-octanol is preferred, and 2-octanol is particularly preferred. The secondary alcohol is more preferably at least one selected from the group consisting of 2-octanol, 3-octanol and 4-octanol, even more preferably 2-octanol, 3-octanol or 4-octanol, and particularly preferably 2-octanol. 2-Octanol is a renewable alcohol, is specifically obtained as a byproduct of sebacic acid obtained by cracking of castor oil, and is useful as bio **1-**methylheptyl (meth)acrylate (bio (meth)acrylic acid ester). Thus, in a preferred embodiment of the present invention, the secondary alcohol is 2-octanol, and, at this time, 1-methylheptyl (meth)acrylate is produced. The 1-methylheptyl (meth)acrylate has the following structure. The 1-methylheptyl (meth)acrylate preferably includes at least one compound selected from the group consisting of 1-methylheptyl acrylate and 1-methylheptyl methacrylate, and more preferably includes 1-methylheptyl methacrylate. The 1-methylheptyl (meth)acrylate is preferably 1-methylheptyl acrylate or 1-methylheptyl methacrylate, particularly preferably 1-methylheptyl methacrylate.

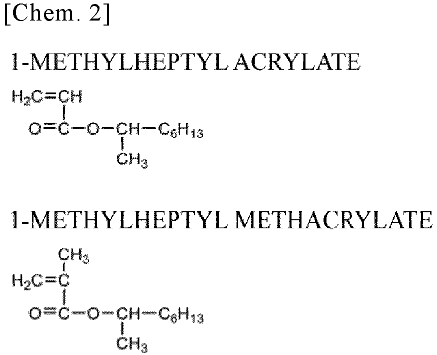

Further, the (meth)acrylic acid ester is obtained by reacting the secondary alcohol with (meth)acrylic acid in equimolar amounts, but the secondary alcohol is preferably used in a larger amount. Specifically, an amount of the secondary alcohol to be added is substantially equal to or more than that of the (meth)acrylic acid, and is, for example, a proportion of 0.9 mol or more and 3.0 mol or less, preferably 1.0 mol or more and 2.0 mol or less per mol of the (meth)acrylic acid. When two or more secondary alcohols are used in combination, the amount of the secondary alcohol to be added refers to a total amount of these secondary alcohols. As illustrated in FIG. 1, a part of the secondary alcohol is directly introduced into the reactor 1 via the pipe 11a. In addition, a part of the secondary alcohol may be introduced into the column top part of the first distillation column 2 via the pipe 11c. Thus, reflux of the first distillation column 2 can be achieved more reliably.

The polymerization inhibitor is not particularly limited, and examples thereof include phenothiazine, hydroquinone, methoquinone, methylhydroquinone, benzoquinone, hydroquinone monomethyl ether, di(tert-butyl)-p-cresol (BHT), p-phenylenediamine, TEMPO (2,2,6,6-tetramethyl-1-piperazinyloxy), p-tert-butylcatechol, di(tert-butyl)catechol, TEMPO derivatives such as OH-TEMPO, 2,6-tert-butyl-4-methylphenol, and copper (II) dibutyldithiocarbamate. These polymerization inhibitors may be used alone, or two or more thereof may be used in combination. The polymerization inhibitor preferably includes at least one compound selected from the group consisting of phenothiazine, hydroquinone, methoquinone, methylhydroquinone, benzoquinone, hydroquinone monomethyl ether, di(tert-butyl)-p-cresol (BHT), p-phenylenediamine, TEMPO (2,2,6,6-tetramethyl-1-piperazinyloxy), p-tert-butylcatechol, di(tert-butyl)catechol, TEMPO derivatives (e.g., OH-TEMPO), 2,6-tert-butyl-4-methylphenol, and copper (II) dibutyldithiocarbamate, and more preferably includes phenothiazine. Among these, phenothiazine is preferred. The polymerization inhibitor is preferably phenothiazine. An amount of the polymerization inhibitor to be added is, for example, a proportion of 0.05 parts by mass or more and 5 parts by mass or less, and preferably 0.1 parts by mass or more and 2 parts by mass or less relative to 100 parts by mass of the (meth)acrylic acid. When two or more polymerization inhibitors are used in combination, the amount of the polymerization inhibitor to be added refers to a total amount of these polymerization inhibitors. The polymerization inhibitor may additionally be added in a subsequent purification treatment step. **In** this case, the amount of the polymerization inhibitor to be added refers to a total amount of the amount thereof initially added to the reactor and the amount thereof additionally added in the subsequent purification treatment step.

The esterification catalyst, (meth)acrylic acid, secondary alcohol and polymerization inhibitor are supplied to the reactor. **In** this case, water formed by the esterification reaction and/or water separately charged into the reactor (water formed by the esterification reaction, water separately charged into the reactor, or a combination thereof) and the secondary alcohol form an azeotropic composition.

Alternatively, an organic solvent may be further supplied to the reactor in addition to the esterification catalyst, the (meth)acrylic acid, the secondary alcohol, and the polymerization inhibitor. In this case, water formed by the esterification reaction and/or water separately charged into the reactor (water formed by the esterification reaction, water separately charged into the reactor, or a combination thereof) and the secondary alcohol and/or the organic solvent if used (the secondary alcohol, the organic solvent if used, or a combination thereof) form an azeotropic composition. Examples of the organic solvent that can be used when the organic solvent is further supplied to the reactor include aliphatic hydrocarbons such as hexane, heptane, pentane, and cyclohexane; aromatic hydrocarbons such as toluene and xylene; ethers such as diethyl ether, diisopropyl ether, methyl-tert-butyl ether, and tetrahydrofuran; and ketones such as acetone, methyl ethyl ketone, diisopropyl ketone, and methyl isobutyl ketone. These organic solvents may be used alone, or two or more thereof may be used in combination. The organic solvent preferably includes at least one solvent selected from the group consisting of aliphatic hydrocarbons, aromatic hydrocarbons, ethers, and ketones, more preferably includes an aromatic hydrocarbon, even more preferably includes at least one solvent selected from the group consisting of toluene and xylene, and particularly preferably includes toluene. Among these, toluene is preferred. The organic solvent is preferably toluene. The organic solvent does not contain any secondary alcohol.

In addition, an amount of the organic solvent to be added is preferably introduced in a proportion of 10 parts by mass or more and 60 parts by mass or less, more preferably 15 parts by mass or more and 40 parts by mass or less, and even more preferably 20 parts by mass or more and 35 parts by mass or less relative to 100 parts by mass of the (meth)acrylic acid. When two or more organic solvents are used in combination, the amount of the organic solvent to be added refers to a total amount of these organic solvents.

The (meth)acrylic acid, secondary alcohol, esterification catalyst, and polymerization inhibitor and, if necessary, organic solvent are supplied to the reactor, and then the (meth)acrylic acid and the secondary alcohol are subjected to a batch reaction in the reactor in the presence of the esterification catalyst. Here, conditions for the batch reaction are not particularly limited, and the same conditions as known ones can be applied. For example, a reaction temperature is preferably 40°C or higher and 120°C or lower, and more preferably 50°C or higher and 110°C or lower. In particular, when the reaction temperature is within the above range, the formation of impurities such as an alkene can be effectively suppressed. In addition, sufficient productivity can be achieved while a reaction rate is secured. A reaction time is preferably 4 hours or more and 24 hours or less, and more preferably 5 hours or more and 15 hours or less, as the reaction time after a predetermined reaction temperature is reached. A pressure at the time of the reaction is not particularly limited and can be appropriately selected from under normal pressure, under reduced pressure, or under increased pressure according to the reaction form. The pressure is preferably under normal pressure or under reduced pressure, more preferably under reduced pressure, and particularly preferably adjusted (reduced) so that the reaction temperature is reached. After the reaction for a predetermined time, the reaction can be terminated by cooling the reactor.

The reactor 1 may be provided with an external heating means (for example, a heat exchanger). In this case, the reactor 1 is heated to a predetermined temperature by the heating means. The reactor 1 may be provided with a stirrer. The esterification reaction is an equilibrium reaction accompanied by generation of water as will be shown below. Therefore, in order to shift the reaction toward the ester formation side, it is necessary to remove the formed water out of the system.

[Chem. 3] CH₂=CH-COOH + R-OH ⇔ CH₂=CH-COOR + H₂O CH₂=C(CH₃)-COOH + R-OH ⇔ CH₂=C(CH₃)-COOR + H₂O

Therefore, the reactor 1 is provided with a distillation column (first distillation column) 2. As a result, the formed water can be distilled off out of the system to remove water from the reaction system. Here, the first distillation column 2 can be, for example, a packed distillation column or plate distillation column having a theoretical plate number of 5 or more and 15 or less (for example, about 10). The reactor 1 and the first distillation column 2 are shown as separate facilities in FIG. 1, but may be integrated into one facility.

As a distillation method in the first distillation column 2, a known method such as simple distillation (for example, flash distillation) or molecular distillation (thin film distillation) can be used, but the method is not particularly limited thereto. At this time, the simple distillation is intended to be batch distillation without a rectifying portion and can be carried out by a general apparatus. The molecular distillation (thin film distillation) can be carried out using a Hickman type distiller, a falling-film type distiller, a rotor tray type distiller, a brush type molecular distiller, or the like. The first distillation column 2 may be heated by a heating means such as a thermosiphon or a forced circulation external heat exchanger. In this case, the first distillation column 2 is heated to a predetermined temperature by the heating means. When the reactor 1 and the first distillation column 2 are provided with an external heating means, the heating means of the reactor 1 and the heating means of the first distillation column 2 may be installed separately or may be the same (shared). Conditions for the distillation are not particularly limited. A distillation pressure is performed under a reduced pressure of, for example, 30 Torr or more and 850 Torr or less, 30 Torr or more and 650 Torr or less (1 Torr = about 1.3 hPa), or the like, or under atmospheric pressure, but is not limited thereto. A distillation temperature (particularly, column bottom temperature) is, for example, 40°C or higher and 120°C or lower, preferably 40°C or higher and 110°C or lower, more preferably 50°C or higher and 100°C or lower, or the like, but is not limited thereto. A distillation time is, for example, 4 hours or more and 24 hours or less, preferably 5 hours or more and 15 hours or less, or the like, but is not limited thereto. Under such conditions, the formed water can be efficiently distilled out of the system.

The water formed by the esterification reaction forms an azeotropic composition with the secondary alcohol and/or the organic solvent if used (the secondary alcohol, the organic solvent if used, or a combination thereof) and is distilled off as a gas from the column top part of the first distillation column 2. This gas is condensed into a liquid in a condenser (not illustrated), charged into an intermediate tank (not illustrated), allowed to stand, and thus separated into an oil phase and an aqueous phase (oil-water separation). A part or all of the oil phase may be charged into the first distillation column 2 as reflux (not illustrated). A part of the oil phase may be supplied (recycled) to the reactor 1 via a pipe 22a and the pipe 11b.

As described above, during the esterification reaction, a mixture containing mainly an azeotropic composition of the secondary alcohol and/or the organic solvent if used (the secondary alcohol, the organic solvent if used, or a combination thereof) and water and a small amount of (meth)acrylic acid is distilled, and the azeotropic composition is condensed, and then separated into two phases, an oil phase and an aqueous phase. To this end, the reactor 1 and the first distillation column (for example, packed distillation column) 2 may be provided with a condenser supplied with water (for example, water at 25°C) and a decanter receiving the condensed azeotropic composition. This decanter may be a decanter equipped with a system which makes it possible to control a lower aqueous phase to a constant position by automatically removing the reaction water formed by opening a solenoid valve. It may be operated under reduced pressure by being adjusted with a vacuum system.

In addition, the aqueous phase part may be partially discarded, or may be partially or entirely supplied to the retention tank 3 via a pipe 22b. The aqueous phase part is not reintroduced into the first distillation column 2. As a result, the equilibrium of the esterification reaction can be constantly shifted toward the (meth)acrylic acid ester formation side (i.e., selectivity and yield can be improved). Alternatively, the secondary alcohol and the (meth)acrylic acid contained at a low concentration may be recovered by subjecting the aqueous phase part to a biological treatment and subjecting the aqueous phase part to a distillation treatment before discharge.

By the esterification reaction, a reaction mixture (reaction mixture A) containing a (meth)acrylic acid ester and an esterified product of the acid-type esterification catalyst as a byproduct is formed in the reactor 1. The reaction mixture A may generally contain the unreacted secondary alcohol, the unreacted (meth)acrylic acid, the esterification catalyst, the polymerization inhibitor, other high-boiling-point byproducts, and the like in addition to the (meth)acrylic acid ester and the esterified product of the acid-type esterification catalyst. The (meth)acrylic acid ester is generally contained in the reaction mixture A in a proportion of about 50 mass% or more and 95 mass% or less.

### Step (b)

In this step, the reaction mixture A obtained in the above step (a) is withdrawn from the bottom of the reactor 1 and supplied to the retention tank 3 via a pipe 31. In addition, the aqueous phase separated from the first distillation column 2 is supplied to the retention tank 3 via the pipe 22b. The reaction mixture A is neutralized and washed with water in the retention tank 3 by supplying the aqueous phase and, if necessary, additional water. Thus, an acid component and an alkali component in the reaction mixture A are removed. In this step, a mixture M1 obtained by neutralizing and water-washing the reaction mixture A is separated into an oil phase O1 and an aqueous phase W1, the aqueous phase W1 is removed from the retention tank 3, and the oil phase O1 is left in the retention tank 3. In the retention tank 3, the oil phase O1 and water are retained while being contacted at a temperature T of 50°C or higher and 105°C or lower. As a result, the esterified product of the esterification catalyst, which has been difficult to remove by neutralization and water washing, is decomposed into the esterification catalyst and the secondary alcohol. Then, the obtained mixture M2 (mixture after warm water treatment) is separated into an oil phase O2 and an aqueous phase W2, and the aqueous phase W2 is removed from the retention tank 3. At this time, since the esterification catalyst is present in the aqueous phase W2, the esterified product of the esterification catalyst in the reaction mixture A is removed in the form of the esterification catalyst. Here, the retention tank 3 may be provided with a stirring device, and may be a tank in which retention is performed while stirring (stirring retention tank) or a mixer. The retention tank 3 may be provided with a heating means.

Neutralization is carried out with a base. Thus, the acid component in the reaction mixture A is neutralized. As the base, for example, sodium hydroxide, potassium hydroxide, sodium carbonate, ammonium carbonate and the like are used. These bases may be used alone, or two or more thereof may be used in combination. The base preferably includes at least one compound selected from the group consisting of sodium hydroxide, potassium hydroxide, sodium carbonate, and ammonium carbonate, and more preferably includes sodium hydroxide. Among these, sodium hydroxide is preferred. Preferably, the base is sodium hydroxide. The base may also be used in the form of an aqueous solution. An amount of the base to be added has only to be an amount that enables neutralization of the residual acid component in the reaction mixture A, and can be appropriately selected in consideration of the amount of the esterification catalyst to be supplied to the reactor, and the like.

After the neutralization (when stirring is performed, the stirring is stopped), the obtained mixture is allowed to stand, and thus separated into an oil phase and an aqueous phase (oil-water separation). The aqueous phase part (the acid component containing the esterification catalyst and the alkali component (base)) is removed out of the system via a pipe 33, and only the oil phase is left in the retention tank 3. (Neutralization step).

Next, water is charged into the retention tank 3, and, if necessary, stirred to remove the base remaining in the oil phase (water washing). After water washing (when stirring is performed, the stirring is stopped), the obtained mixture M1 is allowed to stand to separate the mixture M1 into an oil phase and an aqueous phase (oil-water separation). Among them, the aqueous phase (aqueous phase W1) portion (alkali component (base)) is removed out of the system via the pipe 33, and only the oil phase (oil phase O1) is left in the retention tank 3. (Water washing step).

The neutralization step and the water washing step may be performed as separate steps in a batch operation, or neutralization and water washing may be carried out continuously in a continuous extraction column (neutralization step and water washing step may be carried out continuously in a continuous extraction column). In addition, each of the neutralization step and the water washing step may be performed once or repeatedly.

By these steps, most or all of the esterification catalyst contained in the reaction mixture A is removed out of the system as an aqueous phase.

The neutralization treatment and the water washing treatment are preferably performed at a temperature of lower than 50°C. For example, in the neutralization treatment and the water washing treatment, a liquid temperature of the mixture containing water is preferably lower than 50°C.

After water washing, the oil phase O1 may be heated, and when an organic solvent is used, the oil phase O1 is preferably heated after water washing. After water washing, a heating treatment may be performed on the oil phase O1, and, when an organic solvent is used, a heating treatment is preferably performed on the oil phase O1 after water washing. (Heating step). After water washing, an organic solvent cutting treatment may be performed on the oil phase O1. In the present specification, the organic solvent cutting treatment refers to a treatment of reducing a content of the organic solvent or removing all the organic solvent. The organic solvent cutting treatment may be performed by, for example, distillation of the oil phase O1. A method of the organic solvent cutting treatment is not particularly limited, and examples thereof include a method including heating. Thus, the organic solvent may be recovered as a gas, and may be condensed if necessary. This gas or condensate liquid may be supplied (recycled) to the reactor 1 via a pipe 32 and the pipe 11b. The supply (recycling) of the gas or condensate liquid is generally carried out in batches. Details of a heating method are the same as those in the description of the step (f) (the description of heating of the oil phase after water washing in the step (f)) which will be described below, except that the target for the heating treatment is different. The method of the organic solvent cutting treatment is not particularly limited, and may include, for example, heating and simple distillation of the oil phase O1 in the retention tank 3. A pressure in the retention tank 3 is not particularly limited, but may be gradually reduced, for example. The pressure in the retention tank 3 may be gradually reduced from 680 hPa to 90 hPa, for example. In addition, a temperature of a distillation bottom liquid at this time may be raised, for example, from 110°C to 130°C, and may be raised, for example, from 110°C to 125°C. A removal rate of the organic solvent in the simple distillation is not particularly limited. For example, 90 mass% or more of the organic solvent contained in the bottom liquid is preferably distilled off (removal rate: 90 mass% or more), and 95 mass% or more of the organic solvent is more preferably distilled off (removal rate: 95 mass% or more). The organic solvent cutting treatment is not particularly limited, and examples thereof include a toluene cutting treatment which is a treatment including reducing a content of toluene or completely removing toluene. The organic solvent cutting step in which the organic solvent cutting treatment is performed is not particularly limited, and examples thereof include a toluene cutting step of performing a toluene cutting treatment. If toluene remains in the bottom liquid, it becomes more difficult to lower the pressure (reduce the pressure) in the distillation in the step (c) (distillation in the second distillation column). Therefore, it is preferable to perform the warm water treatment step which will be described below by using the oil phase O1 which has been subjected to the toluene cutting step in which the above-described temperature is used, the above-described removal rate is obtained, or both of them are attained. When the heating treatment, the organic solvent cutting treatment, or a combination thereof is performed after the water washing treatment and before the warm water treatment, hereinafter, the oil phase O1 refers to the oil phase O1 that has undergone the heating treatment, the organic solvent cutting treatment, or a combination thereof.

After water washing (after the water washing treatment), or after the heating treatment, the organic solvent cutting treatment, or a combination thereof, the oil phase O1 and water are retained while being contacted at a temperature T of 50°C or higher and 105°C or lower in the retention tank 3 in which the oil phase O1 is present. As a result, the esterified product of the esterification catalyst, which has been difficult to remove by neutralization and water washing, is decomposed into the esterification catalyst and the secondary alcohol.

The mixture M2 is separated into the oil phase O2 and the aqueous phase M2 (oil-water separation) by retaining the oil phase O1 and water while being contacted at a temperature T of 50°C or higher and 105°C or lower (when stirring is performed, the stirring is stopped), and then allowing the obtained mixture W2 to stand. Among them, a part or all (preferably, all) of the aqueous phase (aqueous phase W2) portion (acid-type esterification catalyst) is removed out of the system via the pipe 33, and the oil phase (oil phase O2) is left in the retention tank 3 (warm water treatment step).

Although moisture may be present in the oil phase (oil phase O2) after the warm water treatment, the moisture will be removed in the same route as the recovered alcohol containing the secondary alcohol (secondary alcohol-rich fluid) in the step (c) which will be described later. Therefore, it is considered that the reduction in yield of the (meth)acrylic acid ester due to mixing of moisture into the oil phase by the warm water treatment hardly occurs.

The oil phase (oil phase O2) after the warm water treatment may be heated. When an organic solvent is used, it is preferable to heat the oil phase (oil phase O2) after the warm water treatment. After water washing, a heating treatment may be performed on the oil phase O2. When an organic solvent is used, it is preferable to perform a heating treatment on the oil phase O2 after water washing. (Heating step). When an organic solvent is used, an organic solvent cutting treatment may be performed on the oil phase (oil phase O2) after the warm water treatment. A method of the organic solvent cutting treatment is not particularly limited, and examples thereof include a method including heating. By these methods, the organic solvent may be recovered as a gas, and may be condensed if necessary. This gas or condensate liquid may be supplied (recycled) to the reactor 1 via the pipe 32 and the pipe 11b. The supply (recycling) of the gas or condensate liquid is generally carried out in batches. Details of a heating method are the same as those in the description of the step (f) (the description of heating of the oil phase after water washing in the step (f)) which will be described below, except that the target for the heating treatment is different. The organic solvent cutting treatment may be performed by, for example, distillation of the oil phase O2. The method of the organic solvent cutting treatment is not particularly limited, and may include, for example, heating and simple distillation of the oil phase O2 in the retention tank 3. A pressure in the retention tank 3 is not particularly limited, but may be gradually reduced, for example. The pressure in the retention tank 3 may be gradually reduced from 680 hPa to 90 hPa, for example. In addition, a temperature of a distillation bottom liquid at this time may be raised, for example, from 110°C to 130°C, and may be raised, for example, from 110°C to 125°C. A removal rate of the organic solvent in the simple distillation is not particularly limited. For example, 90 mass% or more of the organic solvent contained in the bottom liquid is preferably distilled off (removal rate: 90 mass% or more), and 95 mass% or more of the organic solvent is more preferably distilled off (removal rate: 95 mass% or more). The organic solvent cutting treatment is not particularly limited, and examples thereof include a toluene cutting treatment which is a treatment including reducing a content of toluene or completely removing toluene. The organic solvent cutting step in which the organic solvent cutting treatment is performed is not particularly limited, and examples thereof include a toluene cutting step of performing a toluene cutting treatment. If toluene remains in the bottom liquid, it becomes more difficult to lower the pressure (reduce the pressure) in the distillation in the step (c) (distillation in the second distillation column). Therefore, it is preferable to perform, on the oil phase O2, a toluene cutting step in which the above-described temperature is used, the above-described removal rate is obtained, or both of them are attained.

The oil phase (reaction mixture B) containing the (meth)acrylic acid ester remains in the retention tank 3 through the neutralization treatment, the water washing treatment, and the warm water treatment, and, if necessary, further through the heating treatment, the organic solvent cutting treatment, or a combination thereof.

The method of producing a (meth)acrylic acid ester according to a preferred embodiment of the present invention includes contacting a liquid L with water in a reactor and/or a retention tank to separate a mixture containing the liquid L and the water into an organic phase containing a (meth)acrylic acid ester and an aqueous phase containing an acid-type esterification catalyst, and removing a part or all of the aqueous phase.

In the method of producing (meth)acrylic acid according to a preferred embodiment of the present invention, the contact of the liquid L with water in the reactor and/or the retention tank includes using, as the liquid L, a liquid obtained by subjecting a reaction mixture containing a (meth)acrylic acid ester and an esterified product of the esterification catalyst (for example, the reaction mixture A) to a neutralization treatment and a water washing treatment, and contacting the liquid (i.e., the liquid obtained by subjecting the reaction mixture containing the (meth)acrylic acid ester and the esterified product of the esterification catalyst to the neutralization treatment and the water washing treatment) with the water at a temperature T. At this time, it is preferable that the reaction mixture containing the (meth)acrylic acid ester and the esterified product of the esterification catalyst is withdrawn from the reactor and that the liquid L (the liquid obtained by subjecting the reaction mixture to the neutralization treatment and the water washing treatment) is contacted with water in the retention tank. In this embodiment, the liquid L may be a liquid obtained by subjecting a reaction mixture containing a (meth)acrylic acid ester and an esterified product of an esterification catalyst to a neutralization treatment, a water washing treatment, and an organic solvent cutting treatment (for example, a toluene cutting treatment).

In a method of producing (meth)acrylic acid according to a more preferred embodiment of the present invention, the contact of the liquid L with water in the reactor and/or retention tank includes using, as the liquid L, a liquid obtained by subjecting a reaction mixture containing a (meth)acrylic acid ester and an esterified product of the esterification catalyst (for example, the reaction mixture A) to a neutralization treatment and a water washing treatment, and contacting the liquid (i.e., the liquid obtained by subjecting the reaction mixture containing the (meth)acrylic acid ester and the esterified product of the esterification catalyst to the neutralization treatment and the water washing treatment) with the water at a temperature T. The production method further includes supplying a reaction mixture obtained after a treatment including the contact (e.g., the reaction mixture B) to a distillation column and distilling the reaction mixture. At this time, it is preferable that the reaction mixture containing the (meth)acrylic acid ester and the esterified product of the esterification catalyst is withdrawn from the reactor and that the liquid L (i.e., the liquid obtained by subjecting the reaction mixture to the neutralization treatment and the water washing treatment) is contacted with water in the retention tank. In this embodiment, the liquid L may be a liquid obtained by subjecting a reaction mixture containing a (meth)acrylic acid ester and an esterified product of the esterification catalyst to a neutralization treatment, a water washing treatment, and an organic solvent cutting treatment (for example, a toluene cutting treatment).

In the present embodiment (the method of producing a (meth)acrylic acid ester including the above (a), (b), (c), (d), and (e)), the step (a) and the step (b) are performed in different apparatuses as illustrated in FIG. 1. Alternatively, the steps (a) and (b) are carried out in batches. Therefore, as illustrated in FIG. 2, the steps (a) and (b) may be carried out in one apparatus (another embodiment (I)). That is, in the other embodiment (I), the reactor 1 also functions as the retention tank 3 ("1 (3)" in FIG. 2). Specifically, in the other embodiment (I), after completion of the reaction in the above step (a), the aqueous phase or water separated from the first distillation column 2 is supplied to the reaction mixture A in a reactor 1 (3) obtained in the above step (a) via the pipe 22b. In the other embodiment (I), the steps (a) and (b) are carried out in the same manner as the steps (a) and (b), except that the reactor 1 and the retention tank 3 are integrated (the reactor 1 (3) is used). In the case of the other embodiment (I), a tank 25 separately storing the aqueous phase separated from the first distillation column 2 until the aqueous phase is supplied to the reactor 1 (3) may be provided. Alternatively, as illustrated in FIGS. 3 and 4, the aqueous phase separated from the first distillation column 2 may be removed (discharged) via a pipe 22c. In this case, water is separately supplied to perform the step (b) (the neutralization step, the water washing step and the warm water treatment step, and, if necessary, the heating treatment, the organic solvent cutting treatment, or a combination thereof). In FIGS. 3 and 4, the entire amount of the aqueous phase separated from the first distillation column 2 is removed via the pipe 22c, but a part of the aqueous phase may be removed via the pipe 22c and the remainder may be supplied to the retention tank 3 or the reactor 1 (3) (not illustrated). As illustrated in FIGS. 2 and 4, a part or all of the oil phase obtained by condensation and/or oil-water separation of the gas distilled off from the column top part of the first distillation column 2 (the oil phase obtained by condensation of a gas distilled off from the column top part of the first distillation column 2, the oil phase obtained by oil-water separation of a gas distilled off from the column top part of the first distillation column 2 or a condensate liquid thereof, or a combination thereof) may be supplied (recycled) to the reactor 1 (3) via the pipes 22a (32) and 11b.

In the above step (b), an oil phase (reaction mixture B) is obtained in retention tank 3 (FIGS. 1 and 3) or the reactor 1 (3) (FIGS. 2 and 4).

### Step (c)

In this step, the oil phase (reaction mixture B) obtained in the above step (b) is supplied to the second distillation column 4 via a pipe 41 and distilled. As a result, it is separated into a column bottom liquid of the second distillation column 4 containing the (meth)acrylic acid ester and a recovered alcohol containing the secondary alcohol (secondary alcohol-rich fluid) (light-boiling impurity). As described above, the steps (a) and (b) are generally carried out in batches, but the steps subsequent to these steps are carried out continuously (continuous operation).

In the second distillation column 4, the oil phase (reaction mixture B) obtained in the retention tank 3 is distilled. The gas distilled off from the column top or the condensate thereof contains the secondary alcohol, the (meth)acrylic acid and a small amount of the (meth)acrylic acid ester (light-boiling impurity). Therefore, the gas or condensate liquid may be supplied (recycled) to the reactor 1 via a pipe 42 and the pipe 11b. The supply (recycling) of the gas or condensate liquid (recovered alcohol) is generally carried out in batches.

Here, as a distillation method in the second distillation column 4, a known method such as simple distillation (for example, flash distillation) or molecular distillation (thin film distillation) can be used, but the method is not particularly limited thereto. As the second distillation column 4, a packed column or a plate column having a theoretical plate number of 5 or more and 20 or less (for example, theoretical plate number = about 15) can be used. The second distillation column 4 may be heated by a heater such as a thermosiphon or a forced circulation external heat exchanger. Here, conditions for the distillation are not particularly limited. A distillation pressure is performed under a reduced pressure of, for example, 5 Torr or more and 50 Torr or less (1 Torr = about 1.3 hPa) or under atmospheric pressure, but is not limited thereto. A distillation temperature (particularly, column bottom temperature) is, for example, 80°C or higher and 150°C or lower, preferably 100°C or higher and 130°C or lower, but is not limited thereto. Under such conditions, components having a relatively low boiling point (light-boiling impurity containing the secondary alcohol as a main component) can be efficiently distilled off out of the system. If necessary, the polymerization inhibitor may be introduced from the column top part of the second distillation column 4. In this case, the polymerization inhibitor may be used in the form of a solution in which it is dissolved in a secondary alcohol.

A fluid containing a large amount of the (meth)acrylic acid ester (column bottom liquid) remains at the column bottom part of the second distillation column 4 (reaction mixture C).

The operation after the second distillation column 4 is continuous, but, in this case, the recycling (return) of the gas or the condensate thereof (recovered alcohol) is also a batch operation because a batch operation is performed in the return destination. On the other hand, the column bottom liquid of the second distillation column 4 containing the (meth)acrylic acid ester is sent to the third distillation column 5 via a pipe 51.

### Step (d)

In this step, the column bottom liquid (reaction mixture C) of the second distillation column 4 obtained in the above step (c) is withdrawn from the column bottom part of the second distillation column 4, supplied to the third distillation column 5 via the pipe 51, and distilled. As a result, the purified (meth)acrylic acid ester as a final product is distilled off from the top of the distillation column 4 via a pipe 52, and a column bottom liquid (column bottom liquid D) remains in the column bottom part of the third distillation column 5. The purified (meth)acrylic acid ester is usually shipped as a product. On the other hand, the column bottom liquid (column bottom liquid D) of the third distillation column 5 contains high-boiling-point impurities (for example, a Michael adduct generated by addition of a secondary alcohol to a (meth)acrylic acid ester and an ester of a (meth)acrylic acid dimer) and a polymerization inhibitor, and is fed to the next step (treatment apparatus 6) via a pipe 61.

Here, as a distillation method in the third distillation column 5, a known method such as simple distillation (for example, flash distillation) or molecular distillation (thin film distillation) can be used, but the method is not particularly limited thereto. For example, a packed column and a plate column having a theoretical plate number of 5 or more and 20 or less (for example, theoretical plate number = about 5 or more and about 10 or less) can be used. The third distillation column 5 may be heated by a heater such as a thermosiphon or a forced circulation external heat exchanger. Here, conditions for the distillation are not particularly limited. A distillation pressure is performed under a reduced pressure of, for example, 5 Torr or more and 50 Torr or less (1 Torr = about 1.3 hPa) or under atmospheric pressure, but is not limited thereto. A distillation temperature (particularly, column bottom temperature) is, for example, 80°C or higher and 150°C or lower, preferably 100°C or higher and 130°C or lower, but is not limited thereto. Under such conditions, the purified (meth)acrylic acid ester can be efficiently distilled off out of the system. If necessary, the polymerization inhibitor may be introduced from the column top part of the third distillation column 5. In this case, the polymerization inhibitor may be used in the form of a solution in which it is dissolved in a (meth)acrylic acid ester.

In the present embodiment (the method of producing a (meth)acrylic acid ester including the above (a), (b), (c), (d), and (e)), the steps (c) and (d) are performed in different apparatuses as illustrated in FIG. 1, but may be performed in one apparatus as illustrated in FIG. 3 (another embodiment (II)). That is, in the other embodiment (II), the second distillation column 4 and the third distillation column 5 are used as the same apparatus ("4 (5)" in FIG. 3). In the other embodiment (II), the steps (c) and (d) are carried out under the same conditions as those described for the steps (c) and (d) except that they are carried out in one distillation column. Here, when the steps (c) and (d) are carried out in a batch operation, the oil phase (reaction mixture B) obtained in the above step (b) is supplied to the second distillation column 4 (5) via the pipe 41 and distilled (first distillation), and then the column bottom liquid (reaction mixture C) remaining at the bottom is distilled in the second distillation column 4 (5) (second distillation). The gas or condensate liquid (light-boiling impurity) distilled off from the top of the second distillation column 4 (5) in the step (c) (first distillation) is supplied (recycled) to the reactor 1 via the pipes 42 and 11b. Next, in the step (d) (second distillation), the purified (meth)acrylic acid ester as the final product is distilled off from the top of the second distillation column 4 (5) via a pipe 52a by switching a distillation line. Finally, the column bottom liquid (column bottom liquid D) remains at the column bottom part of the second distillation column 4 (5). When the steps (c) and (d) are carried out continuously, the gas or condensate liquid (light-boiling impurity) distilled off from the top of the second distillation column 4 (5) in the step (c) is supplied (recycled) to the reactor 1 via the pipes 42 and 11b, and the purified (meth)acrylic acid ester as the final product is distilled off from the side of the second distillation column 4 (5) via a pipe 52b (side cutting). Finally, the column bottom liquid (column bottom liquid D) remains at the column bottom part of the second distillation column 4 (5).

The embodiments described in the present specification can be combined in any way to constitute another embodiment. For example, as illustrated in FIG. 4, the other embodiment (I) of FIG. 2 and the other embodiment (II) of FIG. 3 may be combined (another embodiment (III)).

### Step (e)

In this step, the column bottom liquid (column bottom liquid D) of the third distillation column 5 obtained in the above step (d) is withdrawn from the column bottom part of the third distillation column 5 and supplied to the treatment apparatus 6 via the pipe 61. As a result, the column bottom liquid (column bottom liquid D) is separated into an active component (recovered (meth)acrylic acid ester) and waste oil.

The treatment apparatus 6 may be a distillation column. Here, as a distillation method in the treatment apparatus 6, a known method such as simple distillation (for example, flash distillation) or molecular distillation (thin film distillation) can be used, but the method is not particularly limited thereto. Preferably, the treatment apparatus 6 is a molecular distillation column (thin film distillation column). Alternatively, the treatment apparatus 6 may be heated by a heater such as a thermosiphon or a forced circulation external heat exchanger. Here, conditions for the distillation are not particularly limited. A distillation pressure is performed under a reduced pressure of, for example, 5 Torr or more and 50 Torr or less (1 Torr = about 1.3 hPa) or under atmospheric pressure, but is not limited thereto. A distillation temperature (particularly, column bottom temperature) is, for example, 80°C or higher and 150°C or lower, preferably 100°C or higher and 130°C or lower, but is not limited thereto. Under such conditions, the active component contained in the column bottom liquid D (hereinafter, also referred to as recovered (meth)acrylic acid ester) and the waste oil can be efficiently separated.

The recovered (meth)acrylic acid ester separated in the treatment apparatus 6 may be supplied (recycled) to the reactor 1 via the pipes 64 and 11b.

The recovered (meth)acrylic acid ester may be supplied (recycled) to the retention tank 3 via a pipe 62. The recovered (meth)acrylic acid ester supplied (recycled) to the retention tank 3 is subjected to the same neutralization step, water washing step and warm water treatment step as in the above step (b), and, if necessary, is further subjected to the heating step, the organic solvent cutting step, the distillation step or a combination thereof. The gas or condensate liquid after these steps may be supplied (recycled) to the reactor 1.

The operation after the second distillation column 4 is continuous, but the recycle (return) of the recovered (meth)acrylic acid ester is also a batch operation because a batch operation is performed in the return destination.

The waste oil separated in the treatment apparatus 6 is discarded via a pipe 63.

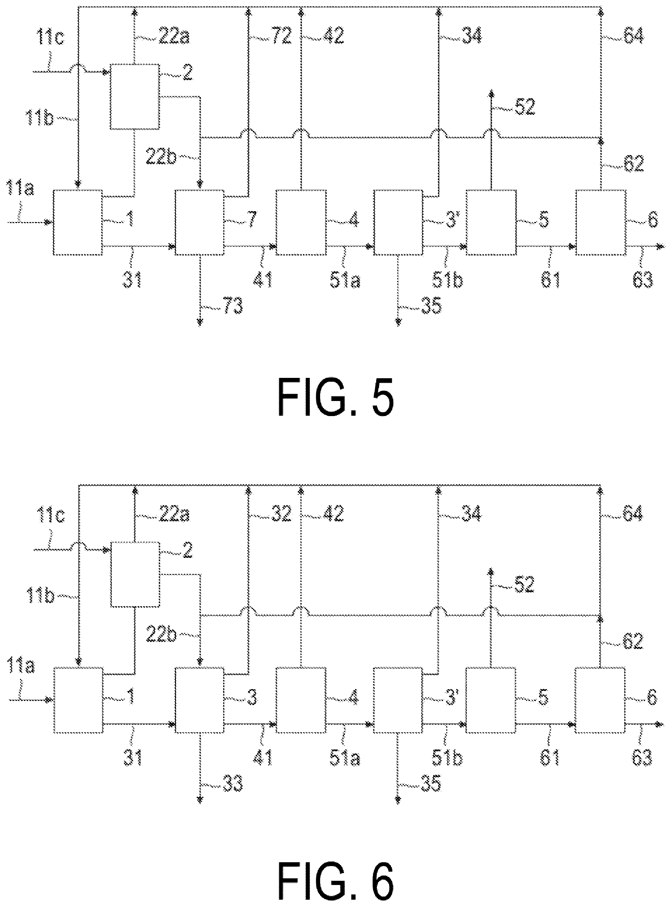

The method of producing a (meth)acrylic acid ester according to another embodiment (IV) of the present invention may further include the following steps (f) and (g) in addition to the above steps (a), (c), (d), and (e). In the other embodiment (IV), the yield of the (meth)acrylic acid ester can be improved when the column bottom liquid of the second distillation column 4 contains the esterified product of the esterification catalyst. A schematic diagram of a production process of the other embodiment (IV) is illustrated as FIG. 5.

The method of producing a (meth)acrylic acid ester according to still another embodiment (V) of the present invention may include the above steps (a), (b), (c), (d), (e), and (g). In the other embodiment (V), the yield of the (meth)acrylic acid ester can be further improved when the column bottom liquid of the second distillation column 4 contains the esterified product of the esterification catalyst. A schematic diagram of a production process of the other embodiment (V) is illustrated as FIG. 6.

(f) After the above step (a) and before the above step (c), withdrawing the reaction mixture A from the bottom of the reactor 1 and supplying it to a neutralization and water washing tank 7, neutralizing and water-washing the reaction mixture A in the neutralization and water washing tank 7, and allowing it to stand for oil-water separation, and then removing an aqueous phase part out of the system via a pipe 73 while obtaining a residue (oil phase part) as a reaction mixture B' after performing a heating treatment, an organic solvent cutting treatment, or a combination thereof, if necessary (for example, if an organic solvent is present, after distilling off the organic solvent by heating, if necessary) (step (f)).

The step (c) after the step (f) is the same as in the above description, except that the reaction mixture B' is used instead of the reaction mixture B in the above description.

(g) After withdrawing the column bottom liquid of the second distillation column 4 from the column bottom part of the second distillation column 4 in the above step (d), supplying it to the third distillation column 5; before distillation, withdrawing the column bottom liquid of the second distillation column 4 from the column bottom part of the second distillation column 4; supplying it to a retention tank 3'; retaining the column bottom liquid (here, the column bottom liquid corresponds to the liquid L since it contains the esterified product of the esterification catalyst) of the second distillation column 4 and water in the retention tank 3' while contacting them at a temperature T of 50°C or higher and 105°C or lower to obtain a mixture M3 (mixture after warm water treatment); separating the mixture M3 into an oil phase O3 and an aqueous phase W3; removing the aqueous phase W3 from the retention tank 3'; optionally recovering the obtained gas or condensate liquid from the retention tank 3'; subjecting a residue (the oil phase O3) in the retention tank 3' to a heating treatment, an organic solvent cutting treatment, or a combination thereof, if necessary; and then supplying it to the third distillation column 5 instead of the column bottom liquid of the second distillation column 4 (step (g)).

### Step (f)

This step is arranged after the step (a) and before the step (c). The step is generally carried out in batches. Details of neutralization and water washing in this step are the same as those in the description of the neutralization and water washing in the step (b). In this step, the reaction mixture A is withdrawn from the bottom of the reactor 1 and supplied to the neutralization and water washing tank 7. The reaction mixture A is neutralized and washed with water in the neutralization and water washing tank 7, and then a heating treatment, an organic solvent cutting treatment or a combination thereof may be performed, if necessary. In this step, the reaction mixture A may be withdrawn from the bottom of the reactor 1 and supplied to the neutralization and water washing tank 7, the reaction mixture A may be neutralized and washed with water in the neutralization and water washing tank 7, and then a heated gas or a condensate liquid thereof may be obtained. After the neutralization, the aqueous phase part (the acid component containing the esterification catalyst and the alkali component (base)) is removed out of the system via the pipe 73, and the oil phase is left in the neutralization and water washing tank 7. Further, in the water washing treatment, the aqueous phase part (alkali component (base)) is removed out of the system via the pipe 73, and the residue (oil phase) in the neutralization and water washing tank 7 is obtained as a reaction mixture B'.

The oil phase after water washing (after water washing treatment) may be heated. When an organic solvent is used, the oil phase after water washing (after water washing treatment) is preferably heated. After water washing, the oil phase may be subjected to a heating treatment. When an organic solvent is used, the oil phase is preferably subjected to a heating treatment after water washing. (Heating step). When an organic solvent is used, the oil phase after water washing (after water washing treatment) may be subjected to an organic solvent cutting treatment. A method of the organic solvent cutting treatment is not particularly limited, and examples thereof include a method including heating. Thus, the organic solvent may be recovered as a gas, and may be condensed if necessary. This gas or condensate liquid may be supplied (recycled) to the reactor 1 via a pipe 72 and the pipe 11b. The supply (recycling) of the gas or condensate liquid is generally carried out in batches.

A method for heating the oil phase after water washing (after water washing treatment) is not particularly limited, and the oil phase may be heated with a heater or distilled. Preferably, the oil phase after water washing is distilled. As a distillation method when distilling the oil phase after water washing, a known method such as simple distillation (for example, flash distillation) or molecular distillation (thin film distillation) can be used, but the method is not particularly limited thereto. For example, a distillation column such as a packed column or plate column having a theoretical plate number of 5 or more and 20 or less can be used. The distillation column may be heated by a heater such as a thermosiphon or a forced circulation external heat exchanger. Here, conditions for the distillation are not particularly limited. A distillation pressure is performed under a reduced pressure of, for example, 5 Torr or more and 50 Torr or less (1 Torr = about 1.3 hPa) or under atmospheric pressure, but is not limited thereto. A distillation temperature (particularly, column bottom temperature) is, for example, 80°C or higher and 150°C or lower, preferably 100°C or higher and 130°C or lower, but is not limited thereto. A distillation time is, for example, 4 hours or more and 24 hours or less, preferably 5 hours or more and 15 hours or less, or the like, but is not limited thereto. Under such conditions, the gas (organic solvent) can be efficiently distilled off out of the system.

The method of the organic solvent cutting treatment is not particularly limited, and may include, for example, heating and simple distillation of the oil phase in the neutralization and water washing tank 7. A pressure in the neutralization and water washing tank 7 is not particularly limited, but may be gradually reduced, for example. The pressure in the neutralization and water washing tank 7 may be gradually reduced from 680 hPa to 90 hPa, for example. In addition, a temperature of a distillation bottom liquid at this time may be raised, for example, from 110°C to 130°C, and may be raised, for example, from 110°C to 125°C. A removal rate of the organic solvent in the simple distillation is not particularly limited. For example, 90 mass% or more of the organic solvent contained in the bottom liquid is preferably distilled off (removal rate: 90 mass% or more), and 95 mass% or more of the organic solvent is more preferably distilled off (removal rate: 95 mass% or more). The organic solvent cutting treatment is not particularly limited, and examples thereof include a toluene cutting treatment which is a treatment including reducing a content of toluene or completely removing toluene. The organic solvent cutting step in which the organic solvent cutting treatment is performed is not particularly limited, and examples thereof include a toluene cutting step of performing a toluene cutting treatment. If toluene remains in the bottom liquid, it becomes more difficult to lower the pressure (reduce the pressure) in the distillation in the step (c) (distillation in the second distillation column). Therefore, it is preferable to perform, on the oil phase in the neutralization and water washing tank 7, a toluene cutting step in which the above-described temperature is used, the above-described removal rate is obtained, or both of them are attained.

An oil phase (reaction mixture B') containing the (meth)acrylic acid ester remains in the neutralization and water washing tank 7 through the neutralization treatment and the water washing treatment in this step, and, if necessary, further through a heating treatment, an organic solvent cutting treatment, or a combination thereof.

Thereafter, in the above step (c), the oil phase (reaction mixture B') obtained in the above step (f) is supplied to the second distillation column 4 via the pipe 41 and distilled. The step (c) after the step (f) is the same as in the above description, except that reaction mixture B' is used instead of the reaction mixture B in the above description.

### Step (g)

This step is carried out after the column bottom liquid of the second distillation column 4 is withdrawn from the column bottom part of the second distillation column 4 and before it is supplied to the third distillation column 5 and distilled, in the step (d). The step is generally carried out in batches. In this step, the column bottom liquid of the second distillation column 4 is withdrawn from the column bottom part of the second distillation column 4 and supplied to the retention tank 3' via the pipe 51a, and the column bottom liquid of the second distillation column 4 and water are retained in the retention tank 3' while being contacted at a temperature T of 50°C or higher and 105°C or lower. As a result, the esterified product of the esterification catalyst, which has been difficult to remove by neutralization and water washing, is decomposed into the esterification catalyst and the secondary alcohol. Then, the obtained mixture M3 (mixture after warm water treatment) is separated into an oil phase O3 and an aqueous phase W3, the aqueous phase W3 is removed from the retention tank 3', and the oil phase (oil phase O3) is left in the retention tank 3'. A part or all (preferably, all) of the aqueous phase (aqueous phase W3) is removed out of the system via a pipe 35. At this time, since the esterification catalyst is present in the aqueous phase W3, the esterified product of the esterification catalyst in the column bottom liquid of the second distillation column 4 is removed in the form of the esterification catalyst (warm water treatment step).

The obtained secondary alcohol is recovered as a gas and condensed if necessary. This gas or condensate liquid may be supplied (recycled) to the reactor 1 via a pipe 34 and the pipe 11b. The supply (recycling) of the gas or condensate liquid is generally carried out in batches.

Here, the retention tank 3' may be provided with a stirring device, and may be a tank in which retention is performed while stirring (stirring retention tank) or a mixer. The retention tank 3' may be provided with a heating means. Details of a warm water treatment in this step are the same as those in the description of the warm water treatment in the step (b) except that the target to be treated and some of the pipes are different.

Although moisture may be present in the oil phase (oil phase O3) after the warm water treatment, the moisture can be removed by side cutting or a divided wall column (DWC) in the step (d) described above. Therefore, it is considered that the reduction in yield of the (meth)acrylic acid ester due to mixing of moisture into the oil phase by the warm water treatment hardly occurs.

The oil phase (oil phase O3) after the warm water treatment may be heated. When an organic solvent is used, it is preferable to heat the oil phase (oil phase O3) after the warm water treatment. After the warm water treatment, a heating treatment may be performed on the oil phase O3. When an organic solvent is used, it is preferable to perform a heating treatment on the oil phase O3 after the warm water treatment. (Heating step). When an organic solvent is used, an organic solvent cutting treatment may be performed on the oil phase (oil phase O3) after the warm water treatment. A method of the organic solvent cutting treatment is not particularly limited, and examples thereof include a method including heating. Thus, the secondary alcohol, the organic solvent or a combination thereof may be recovered as a gas, and may be condensed if necessary. This gas or condensate liquid may be supplied (recycled) to the reactor 1 via the pipes 34 and 11b. The supply (recycling) of the gas or condensate liquid is generally carried out in batches. Details of a heating method are also the same as those in the description of the step of heating the oil phase after water washing in the step (f) (the description of the method for heating the oil phase after water washing) except that the target to be subjected to the heating treatment and some of the pipes are different. The organic solvent cutting treatment may be performed by, for example, distillation of the oil phase O3. The method of the organic solvent cutting treatment is not particularly limited, and may include, for example, heating and simple distillation of the oil phase O3 in the retention tank 3' . A pressure in the retention tank 3' is not particularly limited, but may be gradually reduced, for example. The pressure in the retention tank 3' may be gradually reduced from 680 hPa to 90 hPa, for example. In addition, a temperature of a distillation bottom liquid at this time may be raised, for example, from 110°C to 130°C, and may be raised, for example, from 110°C to 125°C. A removal rate of the organic solvent in the simple distillation is not particularly limited. For example, 90 mass% or more of the organic solvent contained in the bottom liquid is preferably distilled off (removal rate: 90 mass% or more), and 95 mass% or more of the organic solvent is more preferably distilled off (removal rate: 95 mass% or more). The organic solvent cutting treatment is not particularly limited, and examples thereof include a toluene cutting treatment which is a treatment including reducing a content of toluene or completely removing toluene. The organic solvent cutting step in which the organic solvent cutting treatment is performed is not particularly limited, and examples thereof include a toluene cutting step of performing a toluene cutting treatment. If toluene remains in the bottom liquid, it becomes more difficult to lower the pressure (reduce the pressure) in the distillation in the next step. Therefore, it is preferable to perform, on the oil phase O3, a toluene cutting step in which the above-described temperature is used, the above-described removal rate is obtained, or both of them are attained. When the heating treatment, the organic solvent cutting treatment, or a combination thereof is performed after the warm water treatment, hereinafter, the oil phase O3 refers to the oil phase O3 that has undergone the heating treatment, the organic solvent cutting treatment, or a combination thereof.

The residue (oil phase O3) remains in the retention tank 3' through the warm water treatment and, if necessary, a heating treatment, an organic solvent cutting treatment, or a combination thereof. The obtained residue (oil phase O3) in the retention tank 3' is supplied to the third distillation column 5 via a pipe 51b instead of the column bottom liquid of the second distillation column 4.

In the production process of FIG. 5, generally, the above steps (a), (f) and (g) are carried out in batches, and the above steps (c), (d) and (e) are carried out continuously. In this case, an intermediate tank may be provided between the above step (c) and the above step (g) and between the above step (g) and the above step (d).

In the production process of FIG. 6, generally, the above steps (a), (b) and (g) are carried out in batches, and the above steps (c), (d) and (e) are carried out continuously. In this case, an intermediate tank may be provided between the above step (c) and the above step (g) and between the above step (g) and the above step (d).

The method of producing a (meth)acrylic acid ester according to an embodiment of the present invention preferably further includes subjecting a reaction mixture containing the (meth)acrylic acid ester and the esterified product of the esterification catalyst (e.g., the reaction mixture A) to a treatment including a neutralization treatment and a water washing treatment, and distilling a reaction mixture obtained after the treatment including the neutralization treatment and the water washing treatment (e.g., the reaction mixture B or the reaction mixture B') to obtain a post-distillation liquid. The contact of the liquid L with the water in the reactor and/or the retention tank preferably includes using the liquid after distillation as the liquid L, and contacting the liquid after distillation with the water at the temperature T in the retention tank. In this case, the reaction mixture containing the (meth)acrylic acid ester and the esterified product of the esterification catalyst is preferably withdrawn from the reactor. The method of producing a (meth)acrylic acid ester preferably further includes distilling the liquid after the treatment including the contact of the liquid after distillation with water in the retention tank. The treatment including the neutralization treatment and the water washing treatment may be only the neutralization treatment and the water washing treatment, or may further include a treatment other than the neutralization treatment and the water washing treatment in addition to the neutralization treatment and the water washing treatment. The treatment including the neutralization treatment and the water washing treatment may be, for example, a treatment including a neutralization treatment, a water washing treatment, and an organic solvent cutting treatment (for example, a toluene cutting treatment).

The method of producing a (meth)acrylic acid ester according to an embodiment of the present invention more preferably further includes subjecting a reaction mixture containing the (meth)acrylic acid ester and the esterified product of the esterification catalyst (e.g., the reaction mixture A) to a treatment including a neutralization treatment and a water washing treatment, supplying a reaction mixture obtained after the treatment including the neutralization treatment and the water washing treatment (e.g., the reaction mixture B or the reaction mixture B') to a distillation column to distill the reaction mixture, and withdrawing a column bottom liquid of the distillation column from a bottom of the distillation column. The contact of the liquid L with the water in the reactor and/or the retention tank more preferably includes using the column bottom liquid as the liquid L, and contacting the column bottom liquid with the water at the temperature T in the retention tank. In this case, the reaction mixture containing the (meth)acrylic acid ester and the esterified product of the esterification catalyst is preferably withdrawn from the reactor. The method of producing a (meth)acrylic acid ester preferably further includes supplying the column bottom liquid after the treatment including the contact of the column bottom liquid with water in the retention tank to another distillation column and distilling the column bottom liquid. The treatment including the neutralization treatment and the water washing treatment may be only the neutralization treatment and the water washing treatment, or may further include a treatment other than the neutralization treatment and the water washing treatment in addition to the neutralization treatment and the water washing treatment. The treatment including the neutralization treatment and the water washing treatment may be, for example, a treatment including a neutralization treatment, a water washing treatment, and an organic solvent cutting treatment (for example, a toluene cutting treatment).

The method of producing a (meth)acrylic acid ester according to an embodiment of the present invention preferably includes obtaining a liquid L containing an esterified product of an esterification catalyst obtained during production of the (meth)acrylic acid ester, by subjecting a reaction mixture containing a (meth)acrylic acid ester and an esterified product of an esterification catalyst or a liquid obtained through the reaction mixture to an organic solvent cutting treatment (for example, a toluene cutting treatment) before contacting the liquid L with water at a temperature T of 50°C or higher and 105°C or lower in a reactor and/or a retention tank.

The method of producing a (meth)acrylic acid ester according to an embodiment of the present invention particularly preferably satisfies the following (A), (B), and (C):
(A) the contact of the liquid L with water in the reactor and/or the retention tank includes contacting the liquid L with water for a holding time of 3 hours or more and 10 hours or less in a state where the temperature T is held within a range of the set temperature ± 5°C, when the set temperature is a temperature in a range of 95°C or higher and 105°C or lower;
(B) a proportion of a mass of water to a total mass of the liquid L and water to be contacted in the reactor and/or the retention tank is 20 mass% or more and 60 mass% or less; and
(C) the method of producing a (meth)acrylic acid ester includes obtaining the liquid L by subjecting a reaction mixture containing the (meth)acrylic acid ester and the esterified product of the esterification catalyst or a liquid obtained through the reaction mixture to an organic solvent cutting treatment (for example, a toluene cutting treatment) before contacting the liquid L with water at a temperature T in the reactor and/or the retention tank.

In the above (B), the proportion of the mass of water to the total mass of the liquid L and water is preferably 25 mass% or more and 60 mass% or less, more preferably 30 mass% or more and 60 mass% or less, and even more preferably 40 mass% or more and 60 mass%. In the above (B), the proportion of the mass of water to the total mass of the liquid L and water may be 30 mass% or more and 40 mass% or less.

According to the method of producing a (meth)acrylic acid ester of the present invention, a (meth)acrylic acid ester can be produced in a high yield.

The (meth)acrylic acid ester obtained by the method of producing a (meth)acrylic acid ester according to the present invention can be used as a raw material for, for example, a vibration damping material, a printing toner, an ink, a coating material, a pressure-sensitive adhesive, an adhesive, a binder for firing, a synthetic resin, a coating agent, a dispersant, a fiber treatment agent, or the like, but the use of the (meth)acrylic acid ester is not limited thereto.

The embodiments of the present invention have been described in detail, but are illustrative and exemplary and not limitative, and it is apparent that the scope of the present invention should be interpreted based on the appended claims.

The present invention includes the following aspects and forms:
**1. A method** of producing a (meth)acrylic acid ester, including:
   reacting (meth)acrylic acid with a secondary alcohol in a reactor in the presence of an acid-type esterification catalyst and a polymerization inhibitor; and
   contacting a liquid L containing an esterified product of the esterification catalyst obtained during production of the (meth)acrylic acid ester with water at a temperature T of 50°C or higher and 105°C or lower in the reactor and/or a retention tank;
2. The production method according to 1, wherein the contact of the liquid L with the water in the reactor and/or the retention tank includes contacting the liquid L with the water for a holding time of 0.5 hours or more and 100 hours or less in a state where the temperature T is held within a range of a set temperature ± 5°C, when the set temperature is a temperature in a range of 55°C or higher and 100°C or lower;
3. The production method according to 1 or 2, wherein the temperature T is higher than 80°C and 105°C or lower;
4. The production method according to any one of 1 to 3, wherein a proportion of a mass of the water to a total mass of the liquid L and the water is 10 mass% or more;
5. The production method according to any one of 1 to 4, including:
   separating a mixture containing the liquid L and the water into an organic phase containing the (meth)acrylic acid ester and an aqueous phase containing the esterification catalyst by the contact of the liquid L with the water in the reactor and/or the retention tank; and
   removing a part or all of the aqueous phase;
6. The production method according to any one of 1 to 5, wherein
   the contact of the liquid L with the water in the reactor and/or the retention tank includes using, as the liquid L, a liquid obtained by subjecting a reaction mixture containing the (meth)acrylic acid ester and the esterified product to a neutralization treatment and a water washing treatment, and contacting the liquid (that is, liquid obtained through the neutralization treatment and the water washing treatment) with the water at the temperature T;
7. The production method according to any one of 1 to 5, wherein the contact of the liquid L with the water in the reactor and/or the retention tank includes using, as the liquid L, a liquid obtained by subjecting a reaction mixture containing the (meth)acrylic acid ester and the esterified product to a neutralization treatment and a water washing treatment, and contacting the liquid (that is, liquid obtained through the neutralization treatment and the water washing treatment) with the water at the temperature T, and
   the production method further includes supplying a reaction mixture obtained after a treatment including the contact to a distillation column and distilling the reaction mixture;
8. The production method according to any one of 1 to 5, wherein the production method further includes:
   subjecting a reaction mixture containing the (meth)acrylic acid ester and the esterified product to a treatment including a neutralization treatment and a water washing treatment, and distilling a reaction mixture obtained after the treatment including the neutralization treatment and the water washing treatment, and
   the contact of the liquid L with the water in the reactor and/or the retention tank includes using the liquid after distillation as the liquid L, and contacting the liquid after distillation with the water at the temperature T in the retention tank;
9. The production method according to any one of 1 to 5, wherein the production method further includes:
   performing a treatment including a neutralization treatment and a water washing treatment on a reaction mixture containing the (meth)acrylic acid ester and the esterified product;
   supplying a reaction mixture obtained after the treatment including the neutralization treatment and the water washing treatment to a distillation column to perform distillation; and
   withdrawing a column bottom liquid of the distillation column from a bottom of the distillation column, and
   the contact of the liquid L with the water in the reactor and/or the retention tank includes using the column bottom liquid as the liquid L, and contacting the column bottom liquid with the water at the temperature T in the retention tank; and
10. The production method according to any one of 1 to 9, wherein the secondary alcohol is 2-octanol.
11. The production method according to any one of 1 to 10, wherein the acid-type esterification catalyst is a sulfur-containing acid-type esterification catalyst.
12. The production method according to any one of 1 to 11, wherein the (meth)acrylic acid ester has a purity of 99.9 mass% or more.

### Examples

The effects of the present invention will be described with reference to the following examples and comparative examples. However, the technical scope of the present invention is not limited only to the following examples. In the following examples, operations were performed at room temperature (25 ± 5°C), unless otherwise specified. Unless otherwise specified, "%" and "part(s)" mean "mass%" and "part(s) by mass", respectively.

### Example 1-1

### Reaction Step

Into a glass round-bottomed flask having a volume of 5 L and equipped with a stirrer, 1200 g of acrylic acid, 2385 g of 2-octanol (1.1 mol per mol of acrylic acid), 400 g of toluene, 40 g of a 70 mass% aqueous methanesulfonic acid solution, and 6 g of phenothiazine were charged. Thereafter, the round-bottomed flask was immersed in an oil bath to heat the liquid in the round-bottomed flask.

From the time point at which the liquid temperature in the round-bottomed flask reached 110°C as a starting point, the round-bottomed flask was held for 8 hours, and then taken out from the oil bath. Thereafter, the liquid was cooled to terminate the esterification reaction. By this operation, a reaction mixture was obtained. The pressure in the round-bottomed flask was gradually reduced from 800 hPa to 180 hPa so that the liquid temperature in the round-bottomed flask was maintained at 110°C.

After completion of the reaction, the reaction mixture in the round-bottomed flask was analyzed by gas chromatography (GC). As a result, formation of an esterified product (sulfonic acid ester) of an esterification catalyst was observed in addition to 1-methylheptyl acrylate as a reaction product (target product).

### Neutralization Step and Water Washing Step

Subsequently, the reaction mixture obtained in the reaction step was cooled to 40°C, and then 3756 g of the reaction mixture obtained in the reaction step and 1000 g of a 5 mass% aqueous sodium hydroxide solution were charged into a separating funnel having a volume of 5 L, followed by stirring for 5 minutes. Thereafter, the obtained mixture was allowed to stand for oil-water separation, and only the aqueous phase part was withdrawn from the separating funnel.

Thereafter, 1000 g of water was additionally charged into the separating funnel, and stirring was performed for 5 minutes. Thereafter, the obtained mixture was allowed to stand for oil-water separation, and only the aqueous phase part was taken out from the separating funnel.

By the above operation, a liquid (oil phase part) was obtained after completion of the neutralization step and the water washing step. The obtained liquid (oil phase part) was used in the next step (toluene cutting step).

In the neutralization step and the water washing step, the liquid temperature of the mixture containing water was 40°C or lower.

### Toluene Cutting Step

Subsequently, a round-bottomed flask having a volume of 1 L was charged with 900 g of the liquid (oil phase part) obtained after completion of the neutralization step and the water washing step, and then immersed in an oil bath at 130°C to heat the liquid in the round-bottomed flask for simple distillation. The pressure in the round-bottomed flask was gradually reduced from 680 hPa to 90 hPa. The distilling-off ratio in the simple distillation was 15 mass%.

### Warm Water Treatment Step

Subsequently, a glass round-bottomed flask having a volume of 1 L and equipped with a stirrer was charged with 680 g of the liquid (oil phase part) obtained after completion of the toluene cutting step and 120 g of water, and then immersed in an oil bath to heat the liquid in the round-bottomed flask.

The temperature of the oil bath was adjusted so that the set temperature was 100°C. The temperature of a mixture of the reaction mixture in the round-bottomed flask (liquid obtained after completion of the toluene cutting step) and water was held so that the time during which the temperature of the mixture was in the range of 95°C or higher and 105°C or lower was 5 hours, from the time point at which the temperature of the mixture reached 95°C as a starting point. The round-bottomed flask was taken out from the oil bath and cooled.

After cooling, the liquid in the round-bottomed flask was transferred to a separating funnel, allowed to stand for oil-water separation, and then only the oil phase part was taken out from the separating funnel.

By the above operation, a liquid (oil phase part) was obtained after completion of the warm water treatment step.

### Example 1-2

A liquid (oil phase part) was obtained after completion of the warm water treatment step in the same manner as in Example 1-1, except that, in the warm water treatment step, the temperature of the oil bath was changed so that the set temperature was 90°C, and the temperature of a mixture of the reaction mixture in the round-bottomed flask and water was held so that the time during which the temperature of the mixture was in the range of 85°C or higher and 95°C or lower was 21 hours, from the time point at which the temperature of the mixture reached 85°C as a starting point.

### Example 1-3

A liquid (oil phase part) was obtained after completion of the warm water treatment step in the same manner as in Example 1-1, except that, in the warm water treatment step, the temperature of the oil bath was changed so that the set temperature was 80°C, and the temperature of a mixture of the reaction mixture in the round-bottomed flask and water was held so that the time during which the temperature of the mixture was in the range of 75°C or higher and 85°C or lower was 33 hours, from the time point at which the temperature of the mixture reached 75°C as a starting point.

### Example 1-4

A liquid (oil phase part) was obtained after completion of the warm water treatment step in the same manner as in Example 1-1, except that, in the warm water treatment step, the temperature of the oil bath was changed so that the set temperature was 70°C, and the temperature of a mixture of the reaction mixture in the round-bottomed flask and water was held so that the time during which the temperature of the mixture was in the range of 65°C or higher and 75°C or lower was 43 hours, from the time point at which the temperature of the mixture reached 65°C as a starting point.

### Example 1-5

A liquid (oil phase part) was obtained after completion of the warm water treatment step in the same manner as in Example 1-1, except that, in the warm water treatment step, the temperature of the oil bath was changed so that the set temperature was 60°C, and the temperature of a mixture of the reaction mixture in the round-bottomed flask and water was held so that the time during which the temperature of the mixture was in the range of 55°C or higher and 65°C or lower was 62 hours, from the time point at which the temperature of the mixture reached 55°C as a starting point.

### Example 1-6

A liquid (oil phase part) was obtained after completion of the warm water treatment step in the same manner as in Example 1-1, except that, in the warm water treatment step, the temperature of the oil bath was changed so that the set temperature was 50°C, and the temperature of a mixture of the reaction mixture in the round-bottomed flask and water was held so that the time during which the temperature of the mixture was in the range of 45°C or higher and 55°C or lower was 89 hours, from the time point at which the temperature of the mixture reached 45°C as a starting point. In Example 1-6, when the set temperature was set to 50°C in the warm water treatment step and the temperature of the mixture of the reaction mixture and water was held, there was a time during which the temperature was 50°C or higher and 55°C or lower.

### Example 1-7

A liquid (oil phase part) was obtained after completion of the warm water treatment step in the same manner as in Example 1-1, except that, in the warm water treatment step, 720 g of the liquid (oil phase part) obtained after completion of the toluene cutting step and 80 g of water were charged into a glass round-bottomed flask having a volume of 1 L and equipped with a stirrer.

### Example 1-8

A liquid (oil phase part) was obtained after completion of the warm water treatment step in the same manner as in Example 1-1, except that, in the warm water treatment step, 640 g of the liquid (oil phase part) obtained after completion of the toluene cutting step and 160 g of water were charged into a glass round-bottomed flask having a volume of 1 L and equipped with a stirrer.

### Example 1-9

A liquid (oil phase part) was obtained after completion of the warm water treatment step in the same manner as in Example 1-1, except that, in the warm water treatment step, 480 g of the liquid (oil phase part) obtained after completion of the toluene cutting step and 320 g of water were charged into a glass round-bottomed flask having a volume of 1 L and equipped with a stirrer.

### Example 1-10

A liquid (oil phase part) was obtained after completion of the warm water treatment step in the same manner as in Example 1-1, except that, in the warm water treatment step, the temperature of the oil bath was changed so that the set temperature was 90°C, and the temperature of a mixture of the reaction mixture in the round-bottomed flask and water was held so that the time during which the temperature of the mixture was in the range of 85°C or higher and 95°C or lower was 5 hours, from the time point at which the temperature of the mixture reached 85°C as a starting point.

### Example 1-11

A liquid (oil phase part) was obtained after completion of the warm water treatment step in the same manner as in Example 1-1, except that, in the warm water treatment step, the temperature of the oil bath was changed so that the set temperature was 80°C, and the temperature of a mixture of the reaction mixture in the round-bottomed flask and water was held so that the time during which the temperature of the mixture was in the range of 75°C or higher and 85°C or lower was 5 hours, from the time point at which the temperature of the mixture reached 75°C as a starting point.

### Example 1-12

A liquid (oil phase part) was obtained after completion of the warm water treatment step in the same manner as in Example 1-1, except that, in the warm water treatment step, the temperature of the oil bath was changed so that the set temperature was 70°C, and the temperature of a mixture of the reaction mixture in the round-bottomed flask and water was held so that the time during which the temperature of the mixture was in the range of 65°C or higher and 75°C or lower was 5 hours, from the time point at which the temperature of the mixture reached 65°C as a starting point.

### Example 1-13

A liquid (oil phase part) was obtained after completion of the warm water treatment step in the same manner as in Example 1-1, except that, in the warm water treatment step, the temperature of the oil bath was changed so that the set temperature was 60°C, and the temperature of a mixture of the reaction mixture in the round-bottomed flask and water was held so that the time during which the temperature of the mixture was in the range of 55°C or higher and 65°C or lower was 5 hours, from the time point at which the temperature of the mixture reached 55°C as a starting point.

### Example 1-14

A liquid (oil phase part) was obtained after completion of the warm water treatment step in the same manner as in Example 1-1, except that, in the warm water treatment step, the temperature of the oil bath was changed so that the set temperature was 50°C, and the temperature of a mixture of the reaction mixture in the round-bottomed flask and water was held so that the time during which the temperature of the mixture was in the range of 45°C or higher and 55°C or lower was 5 hours, from the time point at which the temperature of the mixture reached 45°C as a starting point. In Example 1-14, when the set temperature was set to 50°C in the warm water treatment step and the temperature of the mixture of the reaction mixture and water was held, there was a time during which the temperature was 50°C or higher and 55°C or lower.

### Example 1-15

A liquid (oil phase part) was obtained after completion of the warm water treatment step in the same manner as in Example 1-1, except that, in the warm water treatment step, the set temperature was set to 100°C, and the temperature of a mixture of the reaction mixture in the round-bottomed flask and water was held so that the time during which the temperature of the mixture was in the range of 95°C or higher and 105°C or lower was 0.1 hours, from the time point at which the temperature of the mixture reached 95°C as a starting point.

### Comparative Example 1-1

A liquid (oil phase part) was obtained after completion of the toluene cutting step in the same manner as in Example 1-1, except that the warm water treatment step was not performed.

### Comparative Example 1-2

A liquid (oil phase part) was obtained after completion of the warm water treatment step in the same manner as in Example 1-1, except that, in the warm water treatment step, the temperature of the oil bath was changed so that the set temperature was 40°C, and the temperature of a mixture of the reaction mixture in the round-bottomed flask and water was held so that the time during which the temperature of the mixture was in the range of 35°C or higher and 45°C or lower was 5 hours, from the time point at which the temperature of the mixture reached 35°C as a starting point. In the warm water treatment step of Comparative Example 1-2, the temperature of the mixture of the reaction mixture and water did not exceed 45°C.

### Comparative Example 1-3

A liquid (oil phase part) was obtained after completion of the warm water treatment step in the same manner as in Example 1-1, except that, in the warm water treatment step, the set temperature was set to 100°C, that only 800 g of the liquid (oil phase part) obtained after completion of the toluene cutting step was charged into a glass round-bottomed flask having a volume of 1 L and equipped with a stirrer, and that the temperature of the reaction mixture in the round-bottomed flask was held so that the time during which the temperature of the mixture was in the range of 95°C or higher and 105°C or lower was 5 hours, from the time point at which the temperature of the mixture reached 95°C as a starting point.

### Example 2-1

### Reaction Step

Into a glass round-bottomed flask having a volume of 5 L and equipped with a stirrer, 1200 g of methacrylic acid, 2000 g of 2-octanol (1.1 mol per mol of methacrylic acid), 400 g of toluene, 80 g of a 70 mass% aqueous methanesulfonic acid solution, and 6 g of phenothiazine were charged. Thereafter, the round-bottomed flask was immersed in an oil bath to heat the liquid in the round-bottomed flask.

From the time point at which the liquid temperature in the round-bottomed flask reached 120°C as a starting point, the round-bottomed flask was held for 9 hours, and then taken out from the oil bath. Thereafter, the liquid was cooled to terminate the esterification reaction. By this operation, a reaction mixture was obtained. The pressure in the round-bottomed flask was gradually reduced from 850 hPa to 330 hPa so that the liquid temperature in the round-bottomed flask was maintained at 120°C.

After completion of the reaction, the reaction mixture in the round-bottomed flask was analyzed by gas chromatography (GC). As a result, formation of an esterified product (sulfonic acid ester) of an esterification catalyst was observed in addition to 1-methylheptyl methacrylate as a reaction product (target product).

### Neutralization Step and Water Washing Step

Subsequently, the reaction mixture obtained in the reaction step was cooled to 40°C, and then 3500 g of the reaction mixture obtained in the reaction step and 1000 g of a 5 mass% aqueous sodium hydroxide solution were charged into a separating funnel having a volume of 5 L, followed by stirring for 5 minutes. Thereafter, the obtained mixture was allowed to stand for oil-water separation, and only the aqueous phase part was withdrawn from the separating funnel.

Thereafter, 1000 g of water was additionally charged into the separating funnel, and stirring was performed for 5 minutes. Thereafter, the obtained mixture was allowed to stand for oil-water separation, and only the aqueous phase part was taken out from the separating funnel.

By the above operation, a liquid (oil phase part) was obtained after completion of the neutralization step and the water washing step. The obtained liquid (oil phase part) was used in the next step (toluene cutting step).

In the neutralization step and the water washing step, the liquid temperature of the mixture containing water was 40°C or lower.

### Toluene Cutting Step

Subsequently, a round-bottomed flask having a volume of 1 L was charged with 900 g of the liquid (oil phase part) obtained after completion of the neutralization step and the water washing step, and then immersed in an oil bath at 130°C to heat the liquid in the round-bottomed flask for simple distillation. The pressure in the round-bottomed flask was gradually reduced from 680 hPa to 90 hPa. The distilling-off ratio in the simple distillation was 20 mass%.

### Warm Water Treatment Step

Subsequently, a glass round-bottomed flask having a volume of 1 L and equipped with a stirrer was charged with 680 g of the liquid (oil phase part) obtained after completion of the toluene cutting step and 120 g of water, and then immersed in an oil bath to heat the liquid in the round-bottomed flask.

The temperature of the oil bath was adjusted so that the set temperature was 100°C. The temperature of a mixture of the reaction mixture in the round-bottomed flask (liquid obtained after completion of the toluene cutting step) and water was held so that the time during which the temperature of the mixture was in the range of 95°C or higher and 105°C or lower was 5 hours, from the time point at which the temperature of the mixture reached 95°C as a starting point. The round-bottomed flask was taken out from the oil bath and cooled.

After cooling, the liquid in the round-bottomed flask was transferred to a separating funnel, allowed to stand for oil-water separation, and then only the oil phase part was taken out from the separating funnel.

By the above operation, a liquid (oil phase part) was obtained after completion of the warm water treatment step.

### Example 2-2

A liquid (oil phase part) was obtained after completion of the warm water treatment step in the same manner as in Example 2-1, except that, in the warm water treatment step, the temperature of the oil bath was changed so that the set temperature was 90°C, and the temperature of a mixture of the reaction mixture in the round-bottomed flask and water was held so that the time during which the temperature of the mixture was in the range of 85°C or higher and 95°C or lower was 21 hours, from the time point at which the temperature of the mixture reached 85°C as a starting point.

### Example 2-3

A liquid (oil phase part) was obtained after completion of the warm water treatment step in the same manner as in Example 2-1, except that, in the warm water treatment step, the temperature of the oil bath was changed so that the set temperature was 80°C, and the temperature of a mixture of the reaction mixture in the round-bottomed flask and water was held so that the time during which the temperature of the mixture was in the range of 75°C or higher and 85°C or lower was 33 hours, from the time point at which the temperature of the mixture reached 75°C as a starting point.

### Example 2-4

A liquid (oil phase part) was obtained after completion of the warm water treatment step in the same manner as in Example 2-1, except that, in the warm water treatment step, the temperature of the oil bath was changed so that the set temperature was 70°C, and the temperature of a mixture of the reaction mixture in the round-bottomed flask and water was held so that the time during which the temperature of the mixture was in the range of 65°C or higher and 75°C or lower was 43 hours, from the time point at which the temperature of the mixture reached 65°C as a starting point.

### Example 2-5

A liquid (oil phase part) was obtained after completion of the warm water treatment step in the same manner as in Example 2-1, except that, in the warm water treatment step, the temperature of the oil bath was changed so that the set temperature was 60°C, and the temperature of a mixture of the reaction mixture in the round-bottomed flask and water was held so that the time during which the temperature of the mixture was in the range of 55°C or higher and 65°C or lower was 62 hours, from the time point at which the temperature of the mixture reached 55°C as a starting point.

### Example 2-6

A liquid (oil phase part) was obtained after completion of the warm water treatment step in the same manner as in Example 2-1, except that, in the warm water treatment step, the temperature of the oil bath was changed so that the set temperature was 50°C, and the temperature of a mixture of the reaction mixture in the round-bottomed flask and water was held so that the time during which the temperature of the mixture was in the range of 45°C or higher and 55°C or lower was 89 hours, from the time point at which the temperature of the mixture reached 45°C as a starting point. In Example 2-6, when the set temperature was set to 50°C in the warm water treatment step and the temperature of the mixture of the reaction mixture and water was held, there was a time during which the temperature was 50°C or higher and 55°C or lower.

### Example 2-7

A liquid (oil phase part) was obtained after completion of the warm water treatment step in the same manner as in Example 2-1, except that, in the warm water treatment step, 680 g of the liquid (oil phase part) obtained after completion of the neutralization step and the water washing step and 120 g of water were charged into a glass round-bottomed flask having a volume of 1 L and equipped with a stirrer, and that the toluene cutting step was performed not before the warm water treatment step but after the warm water treatment step.

### Example 2-8

A liquid (oil phase part) was obtained after completion of the warm water treatment step in the same manner as in Example 2-1, except that, in the warm water treatment step, 560 g of the liquid (oil phase part) obtained after completion of the toluene cutting step and 240 g of water were charged into a glass round-bottomed flask having a volume of 1 L and equipped with a stirrer.

### Example 2-9

A liquid (oil phase part) was obtained after completion of the warm water treatment step in the same manner as in Example 2-1, except that, in the warm water treatment step, 480 g of the liquid (oil phase part) obtained after completion of the toluene cutting step and 320 g of water were charged into a glass round-bottomed flask having a volume of 1 L and equipped with a stirrer.

### Example 2-10

A liquid (oil phase part) was obtained after completion of the warm water treatment step in the same manner as in Example 2-1, except that, in the warm water treatment step, the temperature of the oil bath was changed so that the set temperature was 90°C, and the temperature of a mixture of the reaction mixture in the round-bottomed flask and water was held so that the time during which the temperature of the mixture was in the range of 85°C or higher and 95°C or lower was 5 hours, from the time point at which the temperature of the mixture reached 85°C as a starting point.

### Example 2-11

A liquid (oil phase part) was obtained after completion of the warm water treatment step in the same manner as in Example 2-1, except that, in the warm water treatment step, the temperature of the oil bath was changed so that the set temperature was 80°C, and the temperature of a mixture of the reaction mixture in the round-bottomed flask and water was held so that the time during which the temperature of the mixture was in the range of 75°C or higher and 85°C or lower was 5 hours, from the time point at which the temperature of the mixture reached 75°C as a starting point.

### Example 2-12

A liquid (oil phase part) was obtained after completion of the warm water treatment step in the same manner as in Example 2-1, except that, in the warm water treatment step, the temperature of the oil bath was changed so that the set temperature was 70°C, and the temperature of a mixture of the reaction mixture in the round-bottomed flask and water was held so that the time during which the temperature of the mixture was in the range of 65°C or higher and 75°C or lower was 5 hours, from the time point at which the temperature of the mixture reached 65°C as a starting point.

### Example 2-13

A liquid (oil phase part) was obtained after completion of the warm water treatment step in the same manner as in Example 2-1, except that, in the warm water treatment step, the temperature of the oil bath was changed so that the set temperature was 60°C, and the temperature of a mixture of the reaction mixture in the round-bottomed flask and water was held so that the time during which the temperature of the mixture was in the range of 55°C or higher and 65°C or lower was 5 hours, from the time point at which the temperature of the mixture reached 55°C as a starting point.

### Example 2-14

A liquid (oil phase part) was obtained after completion of the warm water treatment step in the same manner as in Example 2-1, except that, in the warm water treatment step, the temperature of the oil bath was changed so that the set temperature was 50°C, and the temperature of a mixture of the reaction mixture in the round-bottomed flask and water was held so that the time during which the temperature of the mixture was in the range of 45°C or higher and 55°C or lower was 5 hours, from the time point at which the temperature of the mixture reached 45°C as a starting point. In Example 2-14, when the set temperature was set to 50°C in the warm water treatment step and the temperature of the mixture of the reaction mixture and water was held, there was a time during which the temperature was 50°C or higher and 55°C or lower.

### Example 2-15

A liquid (oil phase part) was obtained after completion of the warm water treatment step in the same manner as in Example 2-1, except that, in the warm water treatment step, the set temperature was set to 100°C, and the temperature of a mixture of the reaction mixture in the round-bottomed flask and water was held so that the time during which the temperature of the mixture was in the range of 95°C or higher and 105°C or lower was 0.1 hours, from the time point at which the temperature of the mixture reached 95°C as a starting point.

### Example 2-16

A liquid (oil phase part) was obtained after completion of the warm water treatment step in the same manner as in Example 2-1, except that, in the warm water treatment step, the set temperature was set to 100°C, and the temperature of a mixture of the reaction mixture in the round-bottomed flask and water was held so that the time during which the temperature of the mixture was in the range of 95°C or higher and 105°C or lower was 0.5 hours, from the time point at which the temperature of the mixture reached 95°C as a starting point.

### Comparative Example 2-1

A liquid (oil phase part) was obtained after completion of the toluene cutting step in the same manner as in Example 2-1, except that the warm water treatment step was not performed.

### Comparative Example 2-2

A liquid (oil phase part) was obtained after completion of the warm water treatment step in the same manner as in Example 2-1, except that, in the warm water treatment step, the temperature of the oil bath was changed so that the set temperature was 40°C, and the temperature of a mixture of the reaction mixture in the round-bottomed flask and water was held so that the time during which the temperature of the mixture was in the range of 35°C or higher and 45°C or lower was 5 hours, from the time point at which the temperature of the mixture reached 35°C as a starting point. **In** the warm water treatment step of Comparative Example 2-2, the temperature of the mixture of the reaction mixture and water did not exceed 45°C.

### Comparative Example 2-3

A liquid (oil phase part) was obtained after completion of the warm water treatment step in the same manner as in Example 2-1, except that, in the warm water treatment step, the set temperature was set to 100°C, that only 800 g of the liquid (oil phase part) obtained after completion of the toluene cutting step was charged into a glass round-bottomed flask having a volume of 1 L and equipped with a stirrer, and that the temperature of the reaction mixture in the round-bottomed flask was held so that the time during which the temperature of the mixture was in the range of 95°C or higher and 105°C or lower was 5 hours, from the time point at which the temperature of the mixture reached 95°C as a starting point.

### Evaluation

2-Octanol was quantitatively analyzed according to the following method, for the liquids (oil phase parts) obtained after completion of the warm water treatment step in the Examples, the liquids (oil phase parts) obtained after completion of the toluene cutting step in the Comparative Examples, and the liquids (oil phase parts) obtained after completion of the warm water treatment step in the Comparative Examples.

### Quantitative Analysis of 2-Octanol (Pretreatment)

### Concentration

The liquids (oil phase parts) (100 g) obtained after completion of the warm water treatment step of the Examples were charged into a round-bottomed flask and subjected to simple distillation at an operation pressure of 68 hPa. At a time point when the amount of the distillate reached 75 g, the simple distillation was stopped.

Similarly, 100 g of the liquids (oil phase parts) obtained in the Comparative Examples were charged into a round-bottomed flask and subjected to simple distillation at an operation pressure of 68 hPa. At a time point when the amount of the distillate reached 75 g, the simple distillation was stopped.

### Thermal Aging

The liquid (25 g) remaining in the round-bottomed flask after the concentration was thermally aged at a temperature of 135°C for 6 hours. 2-Octanol was quantitatively analyzed by the following method for the liquid after the thermal aging.

### Quantitative Analysis of 2-Octanol

The concentration (mass%) of 2-octanol was measured by quantitatively analyzing 2-octanol in the liquid using gas chromatography (GC). Conditions for the quantitative analysis were as follows:
Gas chromatography: GC-2014 available from Shimadzu Corporation
Column: DB-1 available from Agilent Technologies, Inc. (column length: 30 m, internal diameter: 0.25 mm, film thickness: 0.25 µm)
Column temperature: 70°C
Carrier gas: He.

The obtained results are shown as "secondary alcohol concentration after thermal aging" in Tables 1 to 4. In this evaluation, a small value of the "secondary alcohol concentration after thermal aging" indicates a high yield of the (meth)acrylic acid ester.

**[Table 1]**

| (Table 1) Conditions for warm water treatment step in production of 1-methylheptyl acrylate and evaluation results | | | | | | | | | | | |
|---|---|---|---|---|---|---|---|---|---|---|---|
| | | Example | Example | Example | Example | Example | Example | Example | Example | Example | Example |
| | | 1-1 | 1-2 | 1-3 | 1-4 | 1-5 | 1-6 | 1-7 | 1-8 | 1-9 | 1-10 |
| Warm water treatment step | Ratio [mass%] of water to total mass of oil phase part and water | 15 | 15 | 15 | 15 | 15 | 15 | 10 | 20 | 40 | 15 |
| | Oil phase part [g] | 680 | 680 | 680 | 680 | 680 | 680 | 720 | 640 | 480 | 680 |
| | Water [g] | 120 | 120 | 120 | 120 | 120 | 120 | 80 | 160 | 320 | 120 |
| | Set temperature [°C] | 100 | 90 | 80 | 70 | 60 | 50 | 100 | 100 | 100 | 90 |
| | Holding time [hour] | 5 | 21 | 33 | 43 | 62 | 89 | 5 | 5 | 5 | 5 |
| Secondary alcohol concentration [mass%] after thermal aging | | 0.50 | 0.50 | 0.50 | 0.50 | 0.50 | 0.50 | 0.56 | 0.46 | 0.37 | 0.71 |

**[Table 2]**

| (Table 2) Conditions for warm water treatment step in production of 1-methylheptyl acrylate and evaluation results | | | | | | | | | |
|---|---|---|---|---|---|---|---|---|---|
| | | Example | Example | Example | Example | Example | Comparative Example | Comparative Example | Comparative Example |
| | | 1-11 | 1-12 | 1-13 | 1-14 | 1-15 | 1-1 | 1-2 | 1-3 |
| Warm water treatment step | Ratio [mass%] of water to total mass of oil phase part and water | 15 | 15 | 15 | 15 | 15 | No warm water treatment step | 15 | 0 |
| | Oil phase part [g] | 680 | 680 | 680 | 680 | 680 | | 680 | 800 |
| | Water [g] | 120 | 120 | 120 | 120 | 120 | | 120 | 0 |
| | Set temperature [°C] | 80 | 70 | 60 | 50 | 100 | | 40 | 100 |
| | Holding time [hour] | 5 | 5 | 5 | 5 | 0.1 | | 5 | 5 |
| Secondary alcohol concentration [mass%] after thermal aging | | 0.97 | 1.07 | 1.11 | 1.15 | 0.93 | 1.18 | 1.17 | 1.34 |

**[Table 3]**

| (Table 3) Conditions for warm water treatment step in production of 1-methylheptyl methacrylate and evaluation results | | | | | | | | | | | |
|---|---|---|---|---|---|---|---|---|---|---|---|
| | | Example | Example | Example | Example | Example | Example | Example | Example | Example | Example |
| | | 2-1 | 2-2 | 2-3 | 2-4 | 2-5 | 2-6 | 2-7 (Note 1) | 2-8 | 2-9 | 2-10 |
| Warm water treatment step | Ratio [mass%] of water to total mass of oil phase part and water | 15 | 15 | 15 | 15 | 15 | 15 | 15 | 30 | 40 | 15 |
| | Oil phase part [g] | 680 | 680 | 680 | 680 | 680 | 680 | 680 | 560 | 480 | 680 |
| | Water [g] | 120 | 120 | 120 | 120 | 120 | 120 | 120 | 240 | 320 | 120 |
| | Set temperature [°C] | 100 | 90 | 80 | 70 | 60 | 50 | 100 | 100 | 100 | 90 |
| | Holding time [hour] | 5 | 21 | 33 | 43 | 62 | 89 | 5 | 5 | 5 | 5 |
| Secondary alcohol concentration [mass%] after thermal aging | | 0.12 | 0.12 | 0.12 | 0.12 | 0.12 | 0.12 | 0.24 | 0.08 | 0.08 | 0.25 |

| | | | | | | | | | | | |
|---|---|---|---|---|---|---|---|---|---|---|---|
| (Note 1) In Example 2-7, the toluene cutting step was not provided after the water washing step and before the warm water treatment step, but was provided after the warm water treatment step. | | | | | | | | | | | |

**[Table 4]**

| (Table 4) Conditions for warm water treatment step in production of 1-methylheptyl methacrylate and evaluation results | | | | | | | | | | |
|---|---|---|---|---|---|---|---|---|---|---|
| | | Example | Example | Example | Example | Example | Example | Comparative Example | Comparative Example | Comparative Example |
| | | 2-11 | 2-12 | 2-13 | 2-14 | 2-15 | 2-16 | 2-1 | 2-2 | 2-3 |
| Warm water treatment step | Ratio [mass%] of water to total mass of oil phase part and water | 15 | 15 | 15 | 15 | 15 | 15 | No warm water treatment step | 15 | 0 |
| | Oil phase part [g] | 680 | 680 | 680 | 680 | 680 | 680 | | 680 | 800 |
| | Water [g] | 120 | 120 | 120 | 120 | 120 | 120 | | 120 | 0 |
| | Set temperature [°C] | 80 | 70 | 60 | 50 | 100 | 100 | | 40 | 100 |
| | Holding time [hour] | 5 | 5 | 5 | 5 | 0.1 | 0.5 | | 5 | 5 |
| Secondary alcohol concentration [mass%] after thermal aging | | 0.39 | 0.44 | 0.47 | 0.48 | 0.48 | 0.41 | 0.49 | 0.53 | 0.49 |

### Example 3-1

The liquid (oil phase part) obtained after completion of the warm water treatment step in Example 1-1 was purified according to the following method to obtain 1-methylheptyl acrylate having a purity of 99.94 mass%.

The liquid (oil phase part) obtained after completion of the warm water treatment step was supplied from the top of a low-boiling-point substance separation column. The low-boiling-point substance separation column was an Oldershaw distillation column having a plate number of 20, and low-boiling-point substances were removed while controlling the operation pressure to a range of from 20 to 30 hPa and the reflux ratio to a range of from 10 to 20 so that the distilling-off ratio was from 5 to 15 mass%. Then, the column bottom liquid of the low-boiling-point substance separation column was supplied from the bottom of a high-boiling-point substance separation column. The high-boiling-point substance separation column was an Oldershaw distillation column having a plate number of 20, and low-boiling-point substances were removed while controlling the operation pressure to a range of from 20 to 30 hPa and the reflux ratio to a range of from 0.1 to 0.5 so that the distilling-off ratio was from 65 to 75 mass%. By the above operation, 1-methylheptyl acrylate (3-1) having a purity of 99.94 mass% was obtained.

### Comparative Example 3-1

The same operation as in Example 3-1 was performed to obtain 1-methylheptyl acrylate (C3-1) having a purity of 99.34 mass%, except that, in Example 3-1, the liquid (oil phase part) obtained in Example 1-1 was changed to the liquid (oil phase part) obtained in Comparative Example 1-1.

### Evaluation

With respect to the 1-methylheptyl acrylate obtained in Example 3-1 and Comparative Example 3-1, the flash point and purity were determined in accordance with the following methods.

### Flash Point

The flash point of the 1-methylheptyl acrylate was determined by the Cleveland open-cup method.

### Purity

The purity of the 1-methylheptyl acrylate was calculated by the following equation. 2-Octene in the 1-methylheptyl acrylate and 2-octanol in the 1-methylheptyl acrylate were subjected to quantitative analysis using gas chromatography (GC) to determine contents (ppm by mass) thereof. An acid component (ppm by mass) in the 1-methylheptyl acrylate was subjected to neutralization titration with 0.1 N-NaOH (quantification as acrylic acid) to determine a content (ppm by mass) thereof. PURITY OF 1-METHYLHEPTYL ACRYLATE (mass%) = 100 - (TOTAL CONTENT OF 2-OCTENE, 2-OCTANOL AND ACID COMPONENT IN 1-METHYLHEPTYL ACRYLATE (mass%))

The evaluation results of the flash point of the 1-methylheptyl acrylate and the purity of the 1-methylheptyl acrylate are shown in Table 5. In Table 5, the flash point of the 1-methylheptyl acrylate is indicated as "flash point", and the purity of the 1-methylheptyl acrylate is indicated as "purity".

**[Table 5]**

| (Table 5) Flash point of 1-methylheptyl acrylate and purity of 1-methylheptyl acrylate | | | | | |
|---|---|---|---|---|---|
| | Purity [mass%] | 2-Octene [ppm by mass] | 2-Octanol [ppm by mass] | Total acid [ppm by mass] | Flash point [°C] |
| Example 3-1 | 99.94 | 55 | 540 | 5 | 94 |
| Comparative Example 3-1 | 99.34 | 2000 | 2500 | 2100 | 83 |

According to the comparison between each Example and each Comparative Example in Tables 1 to 4, the secondary alcohol concentration (mass%) after thermal aging was reduced when the method of producing a (meth)acrylic acid ester included contacting a liquid L containing an esterified product of an esterification catalyst obtained during production of the (meth)acrylic acid ester with water at a temperature T of 50°C or higher and 105°C or lower in a reactor and/or a retention tank. This represents a significant improvement in yield of the (meth)acrylic acid ester.

**In** addition, when the temperature T was higher, the secondary alcohol concentration (mass%) after thermal aging was further reduced. From this fact, it could be confirmed that the (meth)acrylic acid ester was produced in a high yield in a shorter period of time.

Furthermore, by controlling the mass of water relative to the total mass of the liquid L and water, the secondary alcohol concentration (mass%) after thermal aging was further reduced. Thus, it could be confirmed that the (meth)acrylic acid ester was produced in a higher yield.

From the comparison between Example 3-1 and Comparative Example 3-1 in Table 5, it was confirmed that the purity of the (meth)acrylic acid ester was improved when the method of producing a (meth)acrylic acid ester included contacting the liquid L containing the esterified product of the esterification catalyst obtained during the production of the (meth)acrylic acid ester with water at the temperature T of 50°C or higher and 105°C or lower in the reactor and/or the retention tank.

From the comparison between Example 3-1 and Comparative Example 3-1 in Table 5, it was confirmed that the flash point of the 1-methylheptyl acrylate of Example 3-1 subjected to the warm water treatment step was higher than the flash point of the 1-methylheptyl acrylate of Comparative Example 3-1 not subjected to the warm water treatment. This is considered to be because a (meth)acrylic acid ester was produced through the warm water treatment step including contacting the liquid L containing the esterified product of the acid-type esterification catalyst obtained during the production of the (meth)acrylic acid ester with water, whereby low-boiling-point substances (for example, the (meth)acrylic acid and secondary alcohol as raw materials, an alkene as a byproduct, or a combination thereof) were reduced, and a high-purity (meth)acrylic acid ester was obtained.

**In** addition, in the present examples, the production of the (meth)acrylic acid ester was performed using a glass round-bottomed flask equipped with a stirrer or the like, but it is presumed that the effects of the present invention can be similarly obtained when the present invention is applied to a (meth)acrylic acid ester production process as illustrated in FIGS. 1 to 6.

This application is based on Japanese Patent Application No. 2022-185007 filed on November 18, 2022, the disclosure of which is incorporated herein by reference in its entirety.

### Reference Signs List

1: Reactor,
2: First distillation column,
3, 3': Retention tank,
4: Second distillation column,
5: Third distillation column,
6: Treatment apparatus,
7: Neutralization and water washing tank.

## Claims

1. A method of producing a (meth)acrylic acid ester, the method comprising:
reacting (meth)acrylic acid with a secondary alcohol in a reactor in the presence of an acid-type esterification catalyst and a polymerization inhibitor; and
contacting a liquid L containing an esterified product of the esterification catalyst obtained during production of the (meth)acrylic acid ester with water at a temperature T of 50°C or higher and 105°C or lower in the reactor and/or a retention tank.

2. The production method according to claim 1, wherein the contact of the liquid L with the water in the reactor and/or the retention tank comprises contacting the liquid L with the water for a holding time of 0.5 hours or more and 100 hours or less in a state where the temperature T is held within a range of a set temperature ± 5°C, when the set temperature is a temperature in a range of 55°C or higher and 100°C or lower.

3. The production method according to claim 1, wherein the temperature T is higher than 80°C and 105°C or lower.

4. The production method according to claim 1, wherein a proportion of a mass of the water to a total mass of the liquid L and the water is 10 mass% or more.

5. The production method according to claim 1, comprising:
separating a mixture containing the liquid L and the water into an organic phase containing the (meth)acrylic acid ester and an aqueous phase containing the esterification catalyst by the contact of the liquid L with the water in the reactor and/or the retention tank; and
removing a part or all of the aqueous phase.

6. The production method according to claim 1, wherein
the contact of the liquid L with the water in the reactor and/or the retention tank comprises using, as the liquid L, a liquid obtained by subjecting a reaction mixture containing the (meth)acrylic acid ester and the esterified product to a neutralization treatment and a water washing treatment, and contacting the liquid with the water at the temperature T, and
the production method further comprises supplying a reaction mixture obtained after a treatment including the contact to a distillation column to perform distillation.

7. The production method according to claim 1, wherein the production method further comprises:
performing a treatment including a neutralization treatment and a water washing treatment on a reaction mixture containing the (meth)acrylic acid ester and the esterified product;
supplying a reaction mixture obtained after the treatment including the neutralization treatment and the water washing treatment to a distillation column to perform distillation; and
withdrawing a column bottom liquid of the distillation column from a bottom of the distillation column, and
the contact of the liquid L with the water in the reactor and/or the retention tank comprises using the column bottom liquid as the liquid L, and contacting the column bottom liquid with the water at the temperature T in the retention tank.

8. The production method according to claim 1, wherein the secondary alcohol is 2-octanol.

9. The production method according to claim 1, wherein the (meth)acrylic acid ester has a purity of 99.9 mass% or more.
